Europäisches Patentamt

European Patent Office

Office européen des brevets

(11) Publication number: **0 433 345 B1**

# EUROPEAN PATENT SPECIFICATION

(45) Date of publication of patent specification: **20.04.94**  (51) Int. Cl.⁵: **C07K  1/04**, C08F 255/02

(21) Application number: **89910077.0**

(22) Date of filing: **31.08.89**

(86) International application number:
**PCT/DK89/00201**

(87) International publication number:
**WO 90/02749 (22.03.90 90/07)**

(54) **PEPTIDE SYNTHESIS METHOD AND SOLID SUPPORT FOR USE IN THE METHOD.**

(30) Priority: **01.09.88 US 239525**

(43) Date of publication of application:
**26.06.91 Bulletin  91/26**

(45) Publication of the grant of the patent:
**20.04.94 Bulletin  94/16**

(84) Designated Contracting States:
**AT BE CH DE FR GB IT LI LU NL SE**

(56) References cited:
**GB-A- 1 234 982**
**GB-A- 1 344 706**
**US-A- 3 795 664**

(73) Proprietor: **FORSKNINGSCENTER RISO**
**Frederiksborgvej 399**
**P.O. Box 49**
**DK-4000 Roskilde(DK)**

(72) Inventor: **BERG, Rolf, H.**
**Strandvaenget 6**
**DK-2960 Rungsted Kyst(DK)**
Inventor: **ALMDAL, Kristoffer**

**Sporemagervej 16**
**DK-2400 Copenhagen NV(DK)**
Inventor: **PEDERSEN, Walther, Batsberg**
**Rosenhavestraede 4**
**DK-4000 Roskilde(DK)**
Inventor: **HOLM, Arne**
**Margrethevej 19**
**DK-2840 Holte(DK)**
Inventor: **TAM, James, P.**
**500 East 63rd Street, 9J**
**New York, NY 10021(US)**
Inventor: **MERRIFIELD, Robert, Bruce**
**43 Mezzine Drive**
**Cresskil, NJ 07626(US)**

(74) Representative: **Andersen, Henrik Rastrup et al**
**c/o Plougmann & Vingtoft A/S**
**Sankt Annae Plads 11**
**P.O. Box 3007**
**DK-1021 Copenhagen K (DK)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

FIELD OF THE INVENTION

The present invention concerns a method and a solid support for the solid-phase synthesis of peptides or proteins in high yield and in high purity. The method is well suited both to the synthesis of a single peptide or protein and to the parallel and substantially simultaneous synthesis of a multitude thereof. In particular, the invention concerns a method employing a polymer substrate grafted with polystyrene chains as the solid support, the polystyrene chains optionally further bearing substituents which are not reactive under the conditions prevailing in the synthesis, and having an estimated molecular weight, not including optional substituents, of at least 200,000. The invention employs conventional chemical methodology and is readily adapted to both analytical (microgram) and preparative (milligram or larger) scale synthesis. Furthermore, the invention is adaptable to both batchwise and continuous-flow procedures operating manually, semi-automatically or fully automatically.

BACKGROUND OF THE INVENTION

Present day solid-phase methods for the synthesis of peptides or proteins are largely based on the original methodology developed by Merrifield, employing a functionalized cross-linked styrene/divinylbenzene copolymer, the cross-linked copolymer having been formed by the polymerization of styrene monomer to which has been added a few per cent (typically about 2%) of divinylbenzene. This copolymer is generally provided in the form of beads or particles, often with a dominant particle size of 20-80 $\mu$m. The functionalization originally preferred by Merrifield [see e.g. J. Am. Chem. Soc. *85*, 2149 (1963)-] was a functionalization of the aromatic rings of the copolymer with chloromethyl groups, introduced via reaction of the solid copolymer with $SnCl_4$/chloromethyl methyl ether, although a number of other functionalities, including aminomethyl, $\alpha$-aminobenzyl and $\alpha$-amino-4-methylbenzyl, have subsequently been employed. Regardless of its nature, the purpose of the functionality is normally to form an anchoring linkage between the copolymer solid support and the C-terminal of the first amino acid which it is desired to couple to the solid support. More recent refinements of the Merrifield methodology have included the further introduction, between a functionality (e.g. one of the above-mentioned functionalities) on the polystyrene chains and the C-terminal of the first amino acid which is to be coupled, of a bifunctional "spacer" or "handle" group whose reactivity is tailored *inter alia* to meet desired requirements with respect both to the coupling of the first amino acid to the solid support and/or to the ease with which the completed, synthesized peptide or protein chain is cleaved from the solid support. Examples of such spacer groups include the phenylacetamidomethyl (Pam) and the *p*-alkoxybenzyl ester systems. A recent review dealing with the development of solid-phase peptide synthesis methodology since its introduction by Merrifield is given by Barany et al. [Int. J. Peptide Protein Res. *30*, 705-739 (1987)].

GB-A-1,234,982 discloses grafted polystyrene chains of a length much shorter than those obtained according to the present invention.

Recent advances in biotechnology, particularly in the area of recombinant DNA, have produced a unique situation: the availability and rapid accumulation of many new protein sequences with undefined or unknown function and/or unknown biological activity. Detailed structural analysis by site-directed mutagenesis or similar molecular engineering has provided a useful approach concerning the roles of amino acid residues in active sites of proteins.

However, specific information concerning biologically active functional subunits containing ca. 5-40 amino acid residues is preferably obtained through chemical synthesis. Current solid-phase technology is quite sufficient to yield such peptides reliably and in high purity, but the conventional method of solid-phase peptide synthesis via a "linear" mode of approach produces only one peptide per synthesis.

A method employing a "simultaneous" or "parallel" mode of approach to the synthesis of peptides is thus desirable, thereby facilitating the production of a large number of peptides which can, for example, be used to define and map the functional entities of proteins.

A basic feature of the solid-phase technique of peptide synthesis is that in each elongation of the peptide chain with a further amino acid, all treatment steps are repetitive of the previous cycle with the possible exception of the amino acid coupling step itself, in which a further amino acid that may or may not be identical with that coupled in the preceding cycle is coupled to the peptide chain. Thus, a parallel, substantially simultaneous synthesis of more than one peptide can be achieved by performing in parallel the repetitive steps, such as deprotection, neutralization, and washing, which are common to the parallel syntheses. The major technical difficulty is the attainment of compartmentalization of each amino acid coupling step so that cross-contamination will not occur.

Two different methods have recently been proposed for the substantially simultaneous synthesis of a number of peptides:

The first of these methods [Geysen et al., Proc. Natl. Acad. Sci. USA. *81*, 3998-4002 (1984) and *82*, 178-82 (1985)] was devised for rapid screening of peptide epitopes via ELISA (Enzyme Linked Immunosorbent Assay) in 96-microtiter wells. It utilizes acrylic acid-grafted polyethylene rod-and-96-microtiter wells to immobilize growing peptide chains and to perform the compartmentalized synthesis. However, while highly effective, the method is not applicable on a preparative scale, i.e. to the preparation of milligram quantities. The second method [Houghten, Proc. Natl. Acad. Sci. USA. *82*, 5131-35 (1985)] utilizes a "tea bag" containing the traditionally used polymer beads to compartmentalize the synthesis, portions of peptidyl-resin beads being kept apart in sealed bags of fine-mesh polypropylene net. The latter method is relevant to the preparation of milligram quantities.

The obvious advantages of a method permitting the parallel and substantially simultaneous synthesis of a multitude of peptides are the attendant saving in time and the redundancy of the repetitive labour involved in accomplishing the synthesis of each peptide individually.

BRIEF DISCLOSURE OF THE INVENTION

The present invention comprises all the favorable aspects of both of the above-mentioned methods and, in addition, offers the advantages, already mentioned, of providing the desired peptide(s) or protein(s) in high yield and high purity, and being equally well adaptable to both analytical and preparative scale syntheses. The invention will greatly benefit studies in structure-activity relationships, investigations such as mapping of antigenic epitopes, determination of details of hormone-receptor interactions and screening for pharmacologically active peptidyl drugs, as well as being of great value in studies concerning the molecular organization of functional subunits of proteins in general.

The invention is based on the provision and use of a solid support comprising a polymer substrate to which are grafted long and substantially non-cross-linked polystyrene chains which, under these conditions and presumably owing to the easy steric access thereto, function as particularly efficient solid-phase carriers for the peptides to be synthesized.

The invention makes use of the insolubility of non-cross-linked polyolefins, e.g. polyethylene, in all organic solvents at ambient temperature. As the grafted polymer is still a thermoplastic material and soluble at elevated temperatures, reshaping is also possible.

BRIEF DESCRIPTION OF THE FIGURES

Fig. 1. Protection scheme for the solid-phase assembly of [Asp$^{76}$]-hPTH fragment (70-84) on 443 wt % polystyrene-grafted polyethylene.

Fig. 2. Analytical HPLC chromatograms of (A) crude H-Lys-Ala-Lys-Ser-Gln-OH, (B) crude H-Val-Asp-Val-Leu-Thr-Lys-Ala-Lys-Ser-Gln-OH, and (C) crude H-Ala-Asp-Lys-Ala-Asp-Val-Asp-Val-Leu-Thr-Lys-Ala-Lys-Ser-Gln-OH on $\mu$BONDAPAK™ C$_{18}$ (300 x 3.9 mm, 10 $\mu$m). Buffer A: H$_2$O/0.095% CF$_3$COOH; buffer B: 90% acetonitrile/10% H$_2$O/0.072% CF$_3$COOH; flow rate 1.3 ml/min.

Fig. 3. The amino acid sequences of melittin-(7-21) and melittin-(7-21) analogs.

Fig. 4. Analytical HPLC chromatograms of crude melittin-(7-21) and analogs after low/high HF-cleavage (before lyophilization). Chromatogram 1 is that for crude melittin-(7-21), i.e. peptide 1, chromatogram 2 is that for crude peptide 2, etc. Buffer A: 5% CH$_3$CN/95% H$_2$O/0.0445% TFA; buffer B: 60% CH$_3$CN/40% H$_2$O/0.0390% TFA; linear gradient: 5-95% of B in 30 min.; flow rate 1.5 ml/min.; column: Vydac C$_{18}$ (0.46 x 25 cm).

DETAILED DESCRIPTION OF THE INVENTION

In one aspect of the present invention a method for the synthesis of a peptide or a protein is provided, the method comprising the steps of:

A) providing a polymer substrate grafted with polystyrene chains, said polystyrene chains optionally further bearing substituents which are not reactive under the conditions prevailing in the synthesis, the estimated molecular weight of substantially all of the polystyrene chains grafted to the polymer, not including optional substituents, being at least 200,000, at least part of the polystyrene chains of the polystyrene-grafted polymer substrate being functionalized with a chemical functionality facilitating the formation of an anchoring linkage between the polystyrene moiety and an at least *N*-protected and optionally carboxyl terminal derivatized amino acid,

B) coupling an *N*-protected and optionally carboxyl terminal derivatized amino acid to the functionalized polystyrene moiety, said functionality and said *N*-protected and optionally carboxyl terminal derivatized amino acid being adapted to each other such that the anchoring linkage formed can subsequently be cleaved substantially without degradation of the peptide or protein chain which is to be synthesized,

C) removing the *N*-protecting group from an *N*-protected amino or substituted amino group of the coupled and *N*-protected amino acid, such that reaction of the amino or substituted amino group of the coupled amino acid with a carboxyl group or an activated carboxyl group of a further amino acid is facilitated,

D) reacting said amino or substituted amino group of the last-coupled amino acid with a carboxyl group or an activated carboxyl group of a further *N*-protected amino acid so as to form a peptide bond between the two amino acid moieties,

E) optionally removing the *N*-protecting group from an *N*-protected amino or substituted amino group of the last-coupled *N*-protected amino acid, such that reaction of the amino or substituted amino group of the latter amino acid with a carboxyl group or activated carboxyl group of a further *N*-protected amino acid is facilitated,

F) in those cases where step E) has been performed, repeating steps D) and E) a desired number of times,

G) optionally removing some or all protecting groups possibly remaining on the amino acid moieties of the synthesized peptide or protein chain,

H) optionally cleaving the linkage anchoring the synthesized peptide or protein chain to the functionalized polystyrene moiety,

and

I) optionally removing any further undesired group from the synthesized peptide or protein chain.

The term polymer substrate as used in the present context denotes any suitable polymer which may be grafted as described and which in itself is substantially insoluble in and inert towards the reaction media used in the synthesis. Suitable polymers may be selected, for example, from polyamides, such as nylon, polyimides, poly(paraxylylenes), poly(halofluoroalkenes), such as poly(tetrafluoroethylene) or poly-(chlorotrifluoroethylene), phenol-formaldehyde polymers and polyolefins, such as polypropylene and polyethylene. The polymer substrate may be fashioned in any suitable form, for example a sheet, film, bead, pellet, disc, ring, tube, rod or net. In preferred embodiments of methods for peptide or protein synthesis according to the present invention, the polymer substrate is low-density polyethylene in the form of a sheet or film, although experiments indicate (*vide infra*) that high-density polyethylene is also suitable.

The polystyrene chains grafted to the polymer substrate may be chains of polystyrene itself or of polystyrene which has been substituted to some extent with substituents which are not capable of reaction under the conditions prevailing in the synthesis. Such substituents may suitably be, for example, alkyl substituents, such as methyl, ethyl, propyl or butyl, alkoxy substituents, such as methoxy, ethoxy, propoxy and butoxy, or aryloxy substituents, such as phenoxy. The substitution will preferably take the form of substitution in the aromatic rings by one or more substituents, e.g. one or more of the above-mentioned substituents, although substitution at non-aromatic carbon atoms of vinyl group origin may also be envisaged. In preferred embodiments of methods for peptide or protein synthesis according to the present invention, the polymer substrate in these cases being polyethylene, the grafted polystyrene chains are chains of non-substituted polystyrene.

It is believed to be particularly advantageous that the polystyrene chains grafted to the polymer substrate are of a molecular weight, not including optional substituents present on the polystyrene chains, of at least 200,000. In a further aspect of the present invention, polystyrene chains fulfilling this condition may suitably be formed by a substantially radical-initiated reaction between the polymer substrate and optionally substituted styrene monomer present in a solution of the monomer in an organic solvent. Experiments have shown (*vide infra*) that when a polyethylene sheet or film is grafted with polystyrene chains under conditions where the polyethylene sheet or film is immersed in solutions, of varying concentration, of styrene monomer in a solvent such as methanol, the radical-initiated reaction being achieved by $\gamma$-radiation, not only does a grafting reaction occur, but non-grafted (i.e. free) polystyrene chains are formed. While there at present is no obvious, straightforward way of determining accurately the molecular weight of the grafted polystyrene chains themselves, the molecular weight of the non-grafted polystyrene chains formed may readily be determined by, e.g., so-called "size-exclusion chromatography". It has been found that when pure styrene monomer is used, i.e. no methanol is present, the molecular weight of the non-grafted polystyrene chains (denoted hereafter as "homopolymer") occluded in the sheet (and extracted from the sheet with dichloromethane) which are formed under a certain set of $\gamma$-radiation conditions is predominantly about 180,000, and that the predominant molecular weight of the homopolymer increases with increasing methanol content of the styrene monomer/methanol solution; for example, in 70:30 (v/v) methanol/styrene the predominant molecular weight ($M_{peak}$) is about 1,000,000.

Results obtained with polystyrene-grafted polyethylene sheet grafted to various extents give strong indications that the molecular weight of the homopolymer occluded in the sheet and that of the grafted polystyrene chains correspond quite well, as will be explained at greater length in the following:

Size-exclusion chromatography establishes a relationship between species molecular weight and retention volume, the so-called "calibration curve". The molecular weight of a given fraction of, for example, polystyrene homopolymer with a particular retention volume is determined by comparison with retention volumes for polystyrene standards of known molecular weight. However, since no polystyrene-grafted polyethylene standards of known chain molecular weight are available, the best that can be done is to compare with the retention volumes of polystyrene standards under the same solution conditions.

The grafted sheet, the homopolymer and the polystyrene standards may be dissolved, e.g., in hot xylene, and in several such experiments the molecular weight of the most abundant fraction ($M_{peak}$) of homopolymer was found to be ca. 1,000,000. The $M_{peak}$ value found for the polystyrene-grafted polyethylene sheet on the basis of the above-mentioned comparison with retention volumes of polystyrene standards was ca. 3,000,000 and upwards [it can be envisaged that a certain proportion of the individual polyethylene chains may be grafted with more than one polystyrene moiety, and the $M_{peak}$ value determined in the above manner for the polystyrene-grafted polyethylene may consequently be similar to or higher than that for the corresponding occluded polystyrene homopolymer].

Further evidence for the validity of the above-described molecular weight estimation procedure can also be derived from the above-mentioned experiments as follows:

The abundance, $n_i$, of a particular fraction, i, of molecular weight $M_i$ is proportional to the height of the distribution curve at the retention volume corresponding to $M_i$. The so-called "weight average molecular weight", $M_w$, is then given by:

$$M_w = \Sigma\, n_i \times M_i^2\, /\, \Sigma\, n_i \times M_i,$$

while the so-called "number average molecular weight", $M_n$, is given by:

$$M_n = \Sigma\, n_i \times M_i\, /\, \Sigma\, n_i$$

Current theory concerning radical-initiated polymerisation predicts a $M_w/M_n$ ratio (the "polydispersity") of 2.0. A value of ca. 2 was found for the homopolymer, and the value found for the polystyrene-grafted polyethylene was also ca. 2, which may be taken as an indication that the polystyrene chains grafted to the polyethylene substrate have grown in essentially the same manner as the homopolymer, thereby lending further credence to the molecular weight estimation procedure outlined above. The estimated molecular weights referred to in the present description and claims were estimated in the above-described manner.

It is thus believed that the molecular weight of occluded homopolymer formed under a given set of conditions (solvent, styrene concentration, temperature, $\gamma$-radiation intensity and duration of $\gamma$-irradiation) closely reflects the molecular weight of the grafted polystyrene chains formed under the same set of conditions, the molecular weight determined for the homopolymer thus being taken as an estimate of the molecular weight of the grafted polystyrene chains.

It is also believed that the density of grafting sites on the surface of a polymer substrate, i.e. the number of points of attachment of polystyrene chains per unit surface area, as well as the extent of cross-linking of the grafted polystyrene chains, is strongly influenced by the conditions under which grafting takes place, in particular by the nature of an organic solvent used in the grafting process. Hydroxylic organic solvents, particularly alcohols such as methanol, are relatively hydrophilic and are therefore anticipated to be among the poorer solvents which may be chosen to dissolve a relatively hydrophobic substance such as styrene monomer. Thus, the degree of solvation of the monomer by such a solvent is expected to be relatively low by comparison with the degree of solvation which would be expected with a more hydrophobic organic solvent, for example a halogenated aliphatic hydrocarbon such as dichloromethane (dichloromethane being a preferred reaction solvent in solid-phase peptide synthesis methodology, both in general and in the context of the present invention). It is believed that poor swelling or solvation of the grafted polystyrene chains during the grafting process in a solvent such as methanol maintains the mobility of the growing polystyrene chains at a low level thereby leading to retardation of the diffusion-controlled chain-termination processes and thus facilitating the growth of particularly long polystyrene chains.

An attractive feature of the high molecular weight of the grafted polystyrene chains in the context of the present invention is that when functionalized, they may be presumed to behave, with regard to their reactivity towards dissolved reagents, to a large extent as though they were non-grafted (i.e. free) functionalized polystyrene chains in homogeneous solution; the ease with which functionalized, grafted polystyrene chains formed in accordance with the present invention can react with dissolved reagents, including protected and optionally derivatized amino acids, may therefore be regarded as optimal. The apparent substantial absence of cross-linking between the polystyrene chains grafted to the polyolefin facilitates extensive swelling or solvation of the chains by a chlorohydrocarbon solvent (in a preferred embodiment of the present invention dichloromethane) such as generally preferred in solid-phase peptide syntheses. As mentioned previously, in conventional solid-phase peptide synthesis procedures employing "Merrifield-type" methodology, the solid support used is normally a functionalized cross-linked styrene/divinylbenzene copolymer, the cross-linked copolymer having been formed by the polymerization of styrene monomer to which has been added a few per cent (typically ca. 2 %) of divinylbenzene. This cross-linking reduces the degree of swelling or solvation of the functionalized copolymer matrix relative to that prevailing for functionalized, grafted polystyrene chains formed in accordance with the present invention, and thereby correspondingly reduces the reactivity of the former matrix.

According to the invention, it is preferred that the estimated molecular weight of substantially all of the polystyrene chains grafted to the polymer, not including optional substituents, is in the range of 300,000-1,600,000, in particular 400,000-1,400,000, preferably 600,000-1,200,000. The presently preferred estimated molecular weight of substantially all of the polystyrene chains is 700,000-1,000,000. It is believed that the higher estimated molecular weights of 400,000 and above are particularly advantageous, but on the other hand, the grafting of polystyrene chains of the very highest estimated molecular weights of about 1,000,000 and above appears to have a detrimental effect on the mechanical properties of the polymer substrate, in particular when the substrate is, as is often preferred, in the form of a sheet or film.

The degree of polystyrene chain grafting of the polymer substrate, that is, the weight percentage of polystyrene relative to the polymer substrate, depends, of course, on the length of the polystyrene chains, the grafting site density and the dimensions of the polymer substrate, and may vary within wide limits. Thus, in the case of, e.g., a sheet or film of polymer substrate of thickness in the range of 25 to 100 $\mu$m, the degree of polystyrene chain grafting may be, e.g., from about 5 to about 800% by weight, such as from about 10% to about 700%. Both very low and very high degrees of polystyrene chain grafting, as well as intermediate degrees of grafting, are of value in the context of preferred embodiments of the present invention.

Thus, for analytical purposes, where it is normally desired to be able to synthesize peptide sequences of proteins on a small scale, typically microgram scale, the provision, for example, of a polymer substrate with a relatively low degree of polystyrene chain grafting, such as, e.g. 5 to 200%, normally 10 to 60% (for a sheet or film of polymer substrate of thickness in the range of 25 to 100 $\mu$m), and preferably with a controlled, often low extent of functionalization ensures controlled limitation of the amount(s) of peptide(s) formed.

Polystyrene-grafted polyethylene sheet or film produced as preferred within the context of the present invention is particularly advantageous with respect to analytical aspects of peptide chemistry or biochemistry in that its high degree of transparency to light, particularly visible light, facilitates the use of spectrophotometric techniques, e.g. for the monitoring (for example by an ELISA technique) of antigen/antibody reactions which can be monitored by a subsequent colour reaction and in which the antigen may be a particular peptide sequence synthesized on and remaining anchored to the solid support.

For preparative purposes, where the attainment of the highest possible yield of a peptide or protein is clearly desirable, it is advantageous to use the highest practicable degree of grafting. From an overall point of view, the practical upper limit of the degree of grafting for a sheet or film of polymer substrate of thickness in the range of 25 to 100 $\mu$m (as employed according to preferred embodiments) will often be about 500-600% by weight, although special applications may make it desirable to exceed this range, such as to a degree of grafting of about 700%. On the other hand, the lowest degrees of grafting practicable will normally not be below about 40% for such a thin sheet or film. For most practical purposes, the degree of grafting of such a thin sheet or film will be in the range of about 100-600% and will often be in the range of 200-600% and, for preparative purposes, often preferably 200-400% by weight, which seems to be a suitable range both from the point of view of the yield and efficiency of peptide syntheses performed using functionalized, grafted sheet and from the point of view of mechanical strength of the grafted sheet or film.

As will be apparent from the examples illustrating the invention, extraordinarily high yields of highly pure peptides are obtainable, for example, using preferred embodiments of methods according to the present invention which employ functionalized polystyrene-grafted polyethylene sheet or film formed from a polyethylene substrate in the form of a thin sheet or film of thickness in the vicinity of 50 $\mu$m and with a degree of grafting in the vicinity of 400-500%.

As mentioned above, in one aspect of the invention, the polystyrene-grafted polymer substrate is formed by a substantially radical-initiated reaction between the polymer substrate and optionally substituted styrene monomer present in a solution of said monomer in an organic solvent. As also mentioned above, it is advantageous, from the point of view of obtaining long, substantially non-cross-linked polystyrene chains, to perform the grafting in a solvent in which the growing polystyrene chains are poorly swelled or solvated, such as a hydroxylic organic solvent, in particular an alcohol. Preferred alcohols for this purpose are $C_{1-4}$ aliphatic alcohols. In practice, methanol has been found to be a most suitable solvent, but it is contemplated that also, e.g., ethanol, propyl and isopropyl alcohols, and n-butyl, iso-butyl, sec-butyl and tert-butyl alcohols will be applicable.

The volume percentage (% v/v) of optionally substituted styrene in the solution used for the grafting, such as a solution in a solvent which swells or solvates the growing polystyrene chains poorly, e.g. a hydroxylic solvent as explained above, in particular an alcohol as explained above, such as, e.g, methanol, has a marked influence on the molecular weight of the grafted polystyrene chains formed, in that, at least up to a certain point, the chain-length-increasing effect of the solvent is greater, the greater the volume percentage of the solvent in the solution. Thus, while the volume percentage of optionally substituted styrene in the solution may be within a very broad range, such as between 1 and 95%, this volume percentage will normally be in the range of 10 to 90%, more usually 20 to 80%. A very interesting range for the volume percentage of the optionally substituted styrene in the solution is between 25 and 50%, and as will appear from the examples, a range of 25 to 35%, in other words about 30% by volume, has been found in practice to give supports with excellent properties. An indication of the relation between the volume percentage of styrene in methanol during the grafting process and the resulting estimated polystyrene chain lengths appears from the below-mentioned experiments on the relationship between the volume percentage of the optionally substituted styrene in methanol and the molecular weight of the generated homopolymer at a constant $\gamma$-radiation dose and dose-rate.

The grafting process is very suitably performed by $\gamma$-irradiation in the absence of oxygen and at substantially ambient temperature or slightly elevated temperature, the pressure being equal to the total vapour pressure of the liquid components, optionally supplemented by a moderate pressure of an inert gas such as argon, the total pressure then amounting to about 1 atmosphere. A suitable way to remove oxygen from the reaction system is to subject the system to repeated freeze-thaw cycles on a high vacuum apparatus. The $\gamma$-irradiation is suitably performed at a dose rate in the range of from about 1 to about 100,000 Gy/hour, in particular about 200-5000 Gy/hour, such as about 300-1000 Gy/hour. It is believed that the intensity of the irradiation is of considerable importance to the obtainment of the desirable configuration with long, substantially non-cross-linked polystyrene chains; if the intensity is too high, the free radical formation will be so high that the grafting will tend to involve a greater number of shorter chains and perhaps a higher degree of cross-linking, both of which are normally not desired.

On the whole, the optimization of chain length, grafting, and optical properties of the support (which is particularly important when the support is a sheet or film) is performed via the choice of polymer, optionally substituted styrene monomer, reaction mixture, radiation dose-rate, and temperature during irradiation.

While the above-described method involving $\gamma$-irradiation is the presently preferred method, it is contemplated that polystyrene-grafted films may suitably be prepared using a different strategy involving conventional radical initiators, such as peroxides, for example hydrogen peroxide, benzoyl peroxide or diacetyl peroxide, or azo compounds as the radical-forming principles. Other radical-forming principles which may be employed are, e.g., ozone and UV-radiation; another particularly interesting radical-forming principle is an electron beam. The important thing is that the method used for the radical generation be one which is suitable for relatively well-controlled radical-initiated growth of the polystyrene chains. It is believed that the conditions mentioned above concerning the importance of the properties of the solvent used also apply in connection with these free radical initiation principles.

It is also contemplated that it may be possible to produce polystyrene/polyethylene block copolymers useful for the present invention in a manner which does not make use of radical initiation. Thus, for example, it is possible using anionic polymerization to synthesize a block copolymer of butadiene and styrene, in which the chain length of the two blocks can be precisely controlled. It is possible to hydrogenate this polymer in such a manner that the polybutadiene block is converted into polyethylene. The polyethylene formed will have such a regular structure that it, in the solid state, will form high-density polyethylene. It is critical to this method that the polyethylene part of the copolymer should form a coherent film. It is contemplated that this can be obtained in the following manner: the ethylene/styrene block copolymer is dissolved in a solvent in which the polystyrene part is soluble at room temperature and higher temperatures but in which the polyethylene part is only soluble when the solvent is hot. An example of such a solvent is xylene. The polymer solution is placed in a mould and slowly cooled to below the temperature at which polyethylene precipitates. When the polyethylene film has been formed, the rest of the solvent is removed.

It is further contemplated that the latter-outlined alternative method of preparation may be extended to the preparation of other polystyrene/polyolefin block copolymers, for example polystyrene/polypropylene block copolymer, by employing diene monomers other than butadiene, e.g. 2-methyl-1,3-pentadiene in the case of polystyrene/polypropylene block copolymer.

While the polystyrene-grafted polymer substrate may be in any suitable form, such as explained above, very interesting embodiments of the invention are such in which it takes the form of a sheet or film. The thickness of the polymer substrate itself, for example a polyethylene substrate, which is the starting material for such a sheet or film, may vary within a wide range and will normally be from 10 to 10,000 $\mu$m, for most purposes preferably in the range 25 to 1000 $\mu$m, and typically in the range 25 to 100 $\mu$m such as 25 to 75 $\mu$m. The grafting process leads, of course, to an increase in the thickness. Thus, the thinner a sheet or film, the greater will the percentage increase in thickness be for a given set of grafting conditions. As an example, a thin grafted sheet or film may have a thickness in the range of 25 to 200 $\mu$m.

As the grafted polymer in the form of a sheet or film is a thermoplastic material and soluble at elevated temperatures, reshaping after the grafting process is complete is contemplated as a possibility. Thus, in the case of a polystyrene-grafted polyethylene sheet or film, as preferred in the context of the present invention, it is possible to dissolve the sheet or film in a suitable solvent and allow the solution to cool and the solvent to evaporate to obtain a new "casting" of the polymer support, e.g. as a thinner sheet or film, with the grafted polystyrene chains. The suitable solvent is one which, at a suitably high temperature, dissolves the polymer support with its grafted polystyrene chains (but with retention of the grafting) and which on cooling to a lower temperature is no longer capable of retaining the polymer substrate in solution, but still effectively swells or solvates the polystyrene chains. An example of such a solvent, useful, e.g., for polystyrene-grafted polyethylene, is a xylene or a mixture of xylenes.

A sheet or film has a number of advantages in the practical performance of peptide or protein synthesis. Thus, e.g., sheet or film may easily be cut out in suitable sizes for arranging in the reaction vessels used, such as any type of known solid-phase peptide synthesis reaction vessels, including flasks, beakers, microbeakers, funnels, wells, columns or nets.

The film or sheet support makes it possible to devise new practical ways of handling the peptide synthesis. Thus, e.g., a number of sheet or film pieces may be arranged on a common support and thus be kept together during the various stages of peptide synthesis, e.g. by being exposed together to the various reagents and washing solvents, or the pieces may be arranged in sets, each set being subjected to a particular combination of reaction media.

This latter possibility facilitates efficient "compartmentalization" whereby two or more peptides can be prepared in a parallel and substantially simultaneous manner.

Thus, in one aspect the invention provides a particularly practical method for "compartmentalized" synthesis of peptides and proteins, this aspect being based on the use of a polystyrene-grafted sheet or film as the solid-phase peptide synthesis support. This aspect of the invention may be expressed as a method for the synthesis of one or more peptides or proteins, which method, when two or more peptides or proteins are to be synthesized, permits the parallel and substantially simultaneous synthesis of the desired number of peptides and proteins, the method comprising:

a) providing a plurality of substantially identical polymer substrates grafted with polystyrene chains, said polystyrene chains optionally further bearing substituents which are not reactive under the conditions prevailing in the synthesis, at least part of the polystyrene chains of each polystyrene-grafted polymer substrate being functionalized with a chemical functionality facilitating the formation of an anchoring linkage between the polystyrene moieties and an at least *N*-protected and optionally carboxyl terminal derivatized amino acid,

B) optionally physically segregating the members of said plurality of polystyrene-grafted polymer substrates into two or more sets each comprising one or more members of said plurality, coupling an *N*-protected and optionally carboxyl terminal derivatized amino acid to the functionalized polystyrene moieties of each member of said plurality or, where applicable, each member of each set, the *N*-protected and optionally carboxyl terminal derivatized amino acid employed being identical for all the members of the plurality or, where applicable, all the members of one set, and, where applicable, further being in accordance with one of the following alternatives:

(i) identical for all the sets,

(ii) when the number of said sets is greater than two, identical for at least two of the sets,

(iii) different for each set,

said functionality and said *N*-protected and optionally carboxyl terminal derivatized amino acid being adapted to each other such that the anchoring linkage formed can subsequently be cleaved substantially without degradation of the peptide or protein chain which is to be synthesized,

C) treating each member of said plurality or, where applicable, each member of each said set so as to remove the *N*-protecting group from an *N*-protected amino or substituted amino group of the coupled and *N*-protected amino acid, such that reaction of the amino or substituted amino group of the coupled amino acid with a carboxyl group or an activated carboxyl group of a further *N*-protected amino acid is facilitated,

D) reacting said amino or substituted amino group of the amino acid last coupled to the functionalized polystyrene moieties of each member of said plurality or, where applicable, of each member of each set with a carboxyl group or an activated carboxyl group of a further *N*-protected amino acid, so as to form a peptide bond between said amino or substituted amino group and said carboxyl group or activated carboxyl group, said further *N*-protected amino acid being identical for all the members of the plurality or, where applicable, all the members of one set, and, where applicable, further being in accordance with one of the three alternatives mentioned above in connection with step B),

E) optionally treating each member of said plurality or, where applicable, each member of each said set so as to remove the *N*-protecting group from an *N*-protected amino or substituted amino group of the last-coupled *N*-protected amino acid, such that reaction of the amino or substituted amino group of the latter amino acid with a carboxyl group or activated carboxyl group of a further *N*-protected amino acid is facilitated,

F) in those cases where step E) has been performed, repeating steps D) and E) a desired number of times,

G) optionally treating each member of said plurality or, where applicable, each member of each said set so as to remove some or all protecting groups possibly remaining on the amino acid moieties of the synthesized peptide or protein chain,

H) optionally treating each member of said plurality or, where applicable, each member of each said set so as to cleave the linkage anchoring the synthesized peptide or protein chain to the functionalized polystyrene moieties of each member of said plurality or, where applicable, of each member of each said set,

and

I) optionally removing any further undesired group from a synthesized peptide or protein chain.

One practical way of handling the polymer support in the form of a sheet or film for compartmentalization purposes is to cut the sheet or film into a desired number of pieces which are then marked indelibly, e.g. by means of graphite-based ink melted into the surface of some part of the sheet or film. Another possibility is to have the various pieces present on one and the same large piece of sheet or film and then treat the different areas (which are suitably marked as described above) jointly or separately as the case may be. Evidently, one embodiment is to allow the pieces to remain on one and the same film as long as the treatments to be performed are the same, and then divide the film into sub-units when the steps to be performed are different.

The chemical functionality facilitating the formation of an anchoring linkage between an at least *N*-protected and optionally derivatized amino acid and the functionalized polystyrene moiety is suitably a member of, or is derived from a member of the group comprising:

chloro-, bromo- and iodo-substituted alkyl,

amino-substituted alkyl,

amino- and aryl-substituted alkyl,

amino- and alkylaryl-substituted alkyl,

hydroxy-substituted alkyl,

the functionality, if derived from any of said group, being a functionality with a spacer group such that a synthesized peptide or protein chain will be cleavable from the polystyrene moiety substantially without degradation of said chain.

According to suitable embodiments of the invention, chloro-substituted alkyl is chloromethyl, amino-substituted alkyl is aminomethyl, amino- and alkyl-substituted aryl is α-aminobenzyl (benzhydrylamino), amino- and alkylaryl-substituted alkyl is selected from the group consisting of α-amino-2-, α-amino-3- and α-amino-4-methylbenzyl (the latter also being known as 4-methylbenzhydrylamino), and hydroxy-substituted alkyl is hydroxymethyl.

Concerning the initial functionalization of the polystyrene-grafted polymer substrate, more than fifty methods have been described in connection with traditional solid-phase peptide synthesis (see Barany and Merrifield in *The Peptides*, Vol. 2, Academic Press, New York, 1979, pp. 1-284, and Stewart and Young, *Solid Phase Peptide Synthesis*, 2nd Ed., Pierce Chemical Company, Illinois, 1984), of which reactions for the introduction of chloromethyl (via a chloromethyl methyl ether / SnCl₄ reaction, aminomethyl (via a *N*-hydroxymethylphthalimide reaction; see Mitchell et al., *Tetrahedron Lett.*, 3795, (1976)) and benzhydrylamino (Pietta and Marshall, *J. Chem. Soc.*, 650 (1970)) groups are the most widely applied. Other reactive functionalities which have been initially introduced include 4-methylbenzhydrylamino and 4-methoxybenzhydrylamino. All of these established methods are in principle useful within the context of the present invention. Preferred embodiments of peptide or protein synthesis methods within the context of the present invention employ aminomethyl as the initial functionality, in that aminomethyl is particularly advantageous with respect to the incorporation of "spacer" or "handle" groups owing to the reactivity of the amino group of the aminomethyl functionality with respect to the essentially quantitative formation of amide bonds to a carboxylic acid group at one end of the spacer-forming reagent. A vast number of relevant spacer- or handle-forming bifunctional reagents have been described (see Barany et al., *Int. J. Peptide Protein Res.*, 30, 705-739 (1987), especially reagents which are reactive towards amino groups, such as the amino group in the aminomethyl function, including a 4-(haloalkyl)aryl-lower alkanoic acid such as 4-(bromomethyl)phenylacetic acid, a Boc-aminoacyl-4-(oxymethyl)aryl-lower alkanoic acid such as Boc-aminoacyl-4-(oxymethyl)phenylacetic acid, *N*-Boc-*p*-acylbenzhydrylamine such as *N*-Boc-*p*-glutaroylbenzhydrylamine, *N*-Boc-4'-lower alkyl-*p*-acylbenzhydrylamine such as *N*-Boc-4'-methyl-*p*-glutaroylbenzhydrylamine, *N*-Boc-4'-lower alkoxy-*p*-acylbenzhydrylamine such as *N*-Boc-4'-methoxy-*p*-glutaroylbenzhydrylamine and 4-hydroxymethylphenoxy-lower alkanoic acid such as 4-hydroxymethylphenoxyacetic acid.

Certain functionalities, such as benzhydrylamino, 4-methylbenzhydrylamino and 4-methoxybenzhydrylamino which may be incorporated for the purpose of cleavage of a synthesized peptide or protein chain from the solid support such that the C-terminal of the peptide or protein chain is in amide form, require no introduction of a spacer group, and any such functionality may advantageously be employed in the context of the present invention.

An alternative strategy concerning the introduction of spacer or handle groups is the so-called "pre-formed handle" strategy (see Tam et al., *Synthesis*, 955-57, (1979)), which offers complete control over coupling of the first amino acid, and excludes the possibility of complications arising from the presence of undesired functional groups not related to the peptide or protein synthesis. In this strategy, spacer or handle groups, in general spacer or handle groups of the same types as described above, are reacted with the first amino acid which it is desired to anchor to the solid support, the amino acid being *N*-protected and optionally protected at other side-chains which are not relevant with respect to the building-up of the desired peptide or protein chain. Suitable *N*-protecting groups are Boc, normally in combination with benzyl groups for the protection of side chains, and Fmoc, normally in combination with *t*-butyl for the protection of any side chains (Boc = *t*-butyloxycarbonyl; Fmoc = 9-fluorenylmethyloxycarbonyl), although a number of other possibilities exist which are well known in conventional solid-phase peptide synthesis.

Thus, in those cases in which a spacer or handle group is desirable, the first amino acid to be coupled to the solid support can either be coupled to the free reactive end of a spacer group which has been bound to the initially introduced functionality, for example aminomethyl, or can be reacted with the spacer-forming reagent, which in turn is then reacted with the initially introduced functionality.

Following completion of the coupling of the first amino acid which is to be coupled, the next stage of the solid-phase synthesis is the systematic elaboration of the desired peptide or protein chain. This elaboration involves repeated deprotection/coupling cycles. The temporary protecting group, such as a Boc or Fmoc group as described above, on the last-coupled amino acid is quantitatively removed by a suitable treatment, for example by acidolysis, such as with trifluoroacetic acid, in the case of Boc, or by base treatment, such as with piperidine, in the case of Fmoc, so as to liberate the N-terminal amine function of the last-coupled amino acid.

The next desired *N*-protected amino acid is then coupled to the N-terminal of the last-coupled amino acid. This coupling of the C-terminal of an amino acid with the N-terminal of the last-coupled amino acid can be achieved in several ways, for example by providing the incoming amino acid in a form with the carboxyl group activated by any one of several methods, including the initial formation of an active ester derivative, or the initial formation of an anhydride. Alternatively, the carboxyl group of the incoming amino acid may be reacted directly with the N-terminal of the last-coupled amino acid with the assistance of a condensation reagent, for example dicyclohexylcarbodiimide or derivatives thereof.

Following the completed assembly of the desired peptide or protein chain, including protecting groups, the next step will normally be deprotection of the amino acid moieties of the peptide or protein chain and cleavage of the synthesized peptide or protein from the solid support. These processes can take place substantially simultaneously, thereby providing the free peptide in the desired form. Alternatively, in cases in which condensation of two separately synthesized peptide or protein chains is to be carried out, it is possible by choosing a suitable spacer group at the start of the synthesis to cleave the desired peptide or protein chains from their respective solid supports, both peptide or protein chains still incorporating their side-chain protecting groups, and finally removing the side-chain protecting groups after, for example, coupling the two side-chain protected peptide or protein chains to form a longer peptide or protein chain. A third possibility, which is particularly relevant for example in the case of analytical aspects of peptide chemistry or biochemistry, is to remove the side-chain protecting groups from the synthesized peptide or protein chain without cleaving the anchoring linkage holding the chain to the solid support.

It is envisaged that polystyrene-grafted polyethylene substrates analogous to those of the present invention, but comprising linker or spacer groups adapted to the particular chemistry in question, may be valuable in the synthesis of single or multiple biopolymer molecules other than peptides. One example would be the synthesis of oligonucleotides, these being conceptually simple to synthesize since only four different reaction compartments are normally required, one for each of the four nucleotide units involved (i.e. A, T, G and C for DNA fragments, or A, G, C and U for RNA fragments). Such syntheses could be carried out in a parallel and substantially simultaneous fashion, in a manner analogous to that described within the context of the present invention.

The following examples illustrate the invention, the abbreviations used being as follows:

## LIST OF ABBREVIATIONS

| | |
|---|---|
| Boc: | *tert*-butyloxycarbonyl |
| ClZ: | 2-chlorobenzyloxycarbonyl |
| DCC: | N,N'-dicyclohexylcarbodiimide |
| DCU: | N,N'-dicyclohexylurea |
| DIEA: | N,N-diisopropylethylamine |
| DMF: | N,N-dimethylformamide |
| FABMS: | Fast atom bombardment mass spectrometry |
| HOBt: | 1-hydroxybenzotriazole |
| HPLC: | high performance liquid chromatography |
| Pam: | phenylacetamidomethyl |
| PE: | polyethylene |
| PP: | polypropylene |
| SEC: | size-exclusion chromatography |
| SPPS: | solid phase peptide synthesis |
| TFA: | trifluoroacetic acid |
| TFMSA: | trifluoromethanesulfonic acid |
| THF: | tetrahydrofuran |

The abbreviations used for the various amino acids are in accordance with the recommendations of the IUPAC-IUB Commission of Biochemical Nomenclature [J. Biol. Chem., *247*, 977-983 (1972)], and refer in all cases to L-configuration amino acids.

## EXAMPLE 1

*General procedure for preparation of polystyrene−grafted polyethylene sheets.*

Styrene (99% Aldrich) was passed through basic alumina; in some cases it was further distilled from sodium or from calcium hydride. 20 ml of a 30% (v/v) solution of purified styrene in methanol was placed in an ampoule together with a rectangular strip of low-density PE sheet which had been washed in n-hexane. The sheet used had a thickness of 54 $\mu$m. The solution was thoroughly degassed by repeated freeze-thaw cycles on a high vacuum line and the ampoule was then sealed under vacuum. The ampoule and contents were then irradiated in a cobalt gamma-irradiation facility. The irradiation was carried out in two stages, the ampoule being moved from one location in the irradiation source to another between the two stages to ensure as homogeneous a dose distribution as possible. The dose rate was approximately 400 Gy/hour. The highest dose rate used was 417 Gy/hour and the lowest 339 Gy/hour. After irradiation the sheet was extracted in a Soxhlet apparatus with dichloromethane and dried. Specific data are listed in Table 1. It is noteworthy that although there is a clear correlation between the irradiation dose and the extent of the grafting, there is much scatter in the data. This is partly due to the so-called "after effect", the polymerization process continuing to some extent after the irradiation is stopped. As an example of this effect the ampoule containing the sheet irradiated with a total dose of 3.4 kGy to yield a 450 % grafted sheet was left outside the irradiation source for 10 hours between the two stages of irradiation. Furthermore, the ampoule was first opened 10 days after completion of irradiation. A similar procedure was used for the sheet irradiated with a total dose of 2.0 kGy to yield 230% grafting.

TABLE 1

| Grafting of PE sheets in methanol/styrene (70% v/v) | | | |
|---|---|---|---|
| Mass of PE sheet (g) | Irradiation dose (kGy) (Irradiation time in hours) | Graft %* | Duration of extraction with $CH_2Cl_2$ (hours) |
| 0.2288 | 5.6 (14.0) | 547 | 30 |
| 0.290 | 4.0 (10.0) | 443 | 96 |
| 0.3322 | 3.4 (9.0) | 450 | 330 |
| 0.2742 | 3.0 (8.1) | 220 | 240 |
| 0.3866 | 2.9 (8.0) | 285 | 170 |
| 0.3400 | 2.7 (6.7) | 231 | 90 |
| 0.2398 | 2.4 (6.0) | 173 | 120 |
| 0.3399 | 2.0 (6.0) | 230 | 185 |
| 0.3502 | 1.7 (4.8) | 200 | 182 |
| 0.3710 | 1.7 (5.0) | 180 | 260 |
| 0.3456 | 1.0 (3.0) | 75 | 56 |
| 0.3385 | 1.0 (3.0) | 55 | 114[§] |
| 0.3831 | 0.98 (2.8) | 80 | 119 |

* : Graft %  = [(mass of final sheet) -(mass of polyethylene)] x 100/(mass of polyethylene)
§ : The top of the sheet was damaged during the closure of the ampoule.

Sheets with graft % 46, 129 and 331, respectively, have also been prepared.

EXAMPLE 2

*Procedure for grafting on non−woven felt made from fibers consisting of a polypropylene core and another layer of high−density polyethylene.*

The non-woven PP/PE felt was washed with n-hexane and irradiated in a closed ampoule containing a degassed 30% (v/v) solution of purified styrene in methanol in a manner completely analogous to the general procedure described in Example 1. The results are given in Table 2.

TABLE 2

| Grafting of PP/PE non-woven felt in methanol/styrene (70:30 v/v) | | | |
|---|---|---|---|
| Mass of PP/PE felt (g) | Irradiation dose (kGy) (Irradiation time in hours) | Graft %* | Duration of extraction with $CH_2Cl_2$ (hours) |
| 0.2400 | 1.6 (4.5) | 86 | 124 |
| 0.2084 | 2.1 (6.0) | 106 | 72 |

* : Graft %  = [(mass of final sheet) -(mass of polyethylene)] x 100/(mass of polyethylene)

EXAMPLE 3

*Influence of methanol/styrene ratio on grafted polystyrene chain length.*

The following results were obtained for irradiation of low-density polyethylene sheets in different methanol/styrene mixtures (5 kGy dose, 400 Gy/hour dose rate, room temperature):

| % Methanol in solvent (v/v) | Peak molecular weight of homopolymer |
|---|---|
| 0 | 180,000 |
| 20 | 300,000 |
| 40 | 500,000 |
| 70 | 800,000 |

It is seen that the molecular weight (determined by size-exclusion chromatography on cross-linked styrene/divinylbenzene column material) of the homopolymer fraction occluded amongst the grafted chains of the polystyrene-grafted polyethylene sheets and extracted from the sheets with dichloromethane increases as a function of the methanol/styrene ratio in the solution. At the same time the molecular weight distribution tends to become more narrow for high methanol/styrene ratios.

EXAMPLE 4

*Experiments on the estimation of the molecular weight of the grafted polystyrene chains.*

The polystyrene homopolymer extracted from the sheets was characterized by SEC. The extracted polystyrene shows typically a molecular weight distribution with two bulges. This is due to the fact that polystyrene is formed both in the sheet and in the surrounding solution during irradiation. If a sheet is washed briefly in dichloromethane before carrying out Soxhlet extraction, the amount of low molecular weight fraction is greatly reduced relative to that of the high molecular weight fraction. Molecular weight data for the high molecular weight fraction of the extracted homopolymer are given in Table 3. Typical sample sizes were from 0.01 mg to 0.2 mg of polymer. For the homopolymer from the sheets grafted to the extent of 173, 220, 231 and 450 wt %, respectively, a 60 cm column of Toyo Soda TSK GMH6 was used for SEC at ambient temperature with THF as the eluent and a flow rate of 0.5 ml/min. For the homopolymer from the sheet grafted to the extent of 443 wt %, a 50 cm column of Shodex A80-M was used for SEC at 50°C with xylene as the solvent and a flow rate of 0.5 ml/min. This set-up was also used for SEC of the grafted polymer formed, but now operating at 90°C and with a flow rate of approximately 0.3 ml/min. The grafted sheets are soluble in hot xylene. Molecular weight data for polystyrene-grafted polyethylene from the five grafted sheets are given in Table 4. All molecular weight data given were calculated using a calibration curve based on polystyrene standards with molecular weights from 2800 g/mol to 8,000,000 g/mol, the form of the calibration curve being fitted by a third-order polynomial. The use of a first-order (linear) calibration curve leads to similar results. It should be noted that whereas the molecular weights obtained for the styrene homopolymer are absolute, the molecular weights obtained for the graft copolymer are not absolute. In the case of the Shodex A80-M column, extrapolation of the calibration curve was necessary in order to calculate the extremely high molecular weights observed. This extrapolation may lead to underestimation of the weight average molecular weight and consequently also of the polydispersity. The ungrafted polyethylene was characterized by high-temperature SEC using 1,2,4-trichlorobenzene as the eluent. The following values were obtained: $M_w = 4 \times 10^4$ g/mol and $M_w/M_n = 5$. The latter data were obtained using a calibration curve based on polystyrene standards.

The data for the polystyrene homopolymers indicate that the molecular weight is insensitive to the total radiation dose, whereas for the polystyrene-grafted polyethylene the measured molecular weight is largely proportional to the dose. These observations indicate that the very long chain grafts are formed during the entire irradiation process and that essentially only the number of grafts is affected by the dose.

TABLE 3

| Molecular weight data for the high molecular weight fraction of the polystyrene homopolymer extracted from the irradiated sheets | | | |
|---|---|---|---|
| Graft % | Irradiation dose (kGy) | $M_w$[#] $(\times 10^{-6}$ mol/g) | $M_w/M_n$[§] |
| 450 | 3.4 | 1.4 | 2.2 |
| 443 | 4.0 | 2.6 | 3.7 |
| 231 | 2.7 | 1.2 | 2.3 |
| 220 | 3.0 | 1.2 | 2.2 |
| 173 | 2.4 | 1.1 | 2.2 |

\# : $M_w$ = weight average molecular weight

§ : $M_w/M_n$ = weight average molecular weight divided by number average molecular weight

TABLE 4

Molecular weight data for the polystyrene-grafted polyethylene from the irradiated sheets.

| Graft % | Irradiation dose (kGy) | $M_w$[#] $(\times 10^{-6}$ mol/g) | $M_w/M_n$[§] | $M_{peak}$[*] $(\times 10^{-6}$ mol/g) |
|---|---|---|---|---|
| 450 | 3.4 | 7.0 | 1.6 | 7.2 |
| 443 | 4.0 | 6.6 | 1.8 | 6.1 |
| 231 | 2.7 | 2.9 | 2.2 | 3.5 |
| 220 | 3.0 | 4.7 | 1.6 | 4.5 |
| 173 | 2.4 | 4.1 | 1.6 | 3.1 |

\# : $M_w$ = weight average molecular weight

§ : $M_w/M_n$ = weight average molecular weight divided by number average molecular weight

\* : $M_{peak}$ = molecular weight at the peak point in the chromatogram

EXAMPLE 5

*Reshaping of a polystyrene – grafted polyethylene sheet.*

A piece of 173% graft sheet (cf. Table 1) was dissolved in xylene at 100°C and the solution poured into a teflon mold at 80°C. After slow evaporation of the xylene a very thin film was formed. Because of the extreme thinness of the film it was not possible to obtain anything but small pieces (1 to 2 mm square) of film. However, these small pieces did not disintegrate when exposed to dichloromethane. This implies that a continuous polyethylene phase is reformed.

EXAMPLE 6

*Aminomethylation (functionalization) of polystyrene – grafted PE sheets.*

Eight equally sized rectangular strips (1.5 x 4.5 cm) of 443% polystyrene-grafted polyethylene sheet (1.30 g total) were placed in a 60 ml SPPS reaction vessel on a manual SPPS shaker and washed with 40 ml of TFA/$CH_2Cl_2$ (1:1 v/v) for 3 x 5 min. A solution of 0.35 g (1.9 mmol) *N*-(hydroxymethyl)phthalimide (97% purity; EGA-CHEMIE) in 40 ml of TFA/$CH_2Cl_2$ (1:1 v/v) was added to the washed sheets and the mixture was shaken for 10 min. 10 ml of TFMSA/TFA/$CH_2Cl_2$ (10:45:45 v/v/v) was added slowly over a 4-5 hour period and shaking was continued for another 3 hours. The sheets were isolated by filtration and washed sequentially with the following: TFA/$CH_2Cl_2$ (1:1 v/v) (120 ml), $CH_2Cl_2$ (240 ml), methanol (160 ml), and ethanol (160 ml). They were then shaken in 40 ml of ethanol containing 10% of hydrazine (Fluka) for 12 hours at 70°C. The sheets were filtered from the hot mixture and washed sequentially (with 20 min shaking for each wash) with the following: hot ethanol (3 x 40 ml), hot DMF (3 x 40 ml), hot ethanol (3 x 40 ml), hot methanol (3 x 40 ml), and $CH_2Cl_2$ (3 x 40 ml). Finally, the sheets were treated with 40 ml of DIEA/$CH_2Cl_2$ - (1:9 v/v) for 2 x 5 min, washed with 200 ml of $CH_2Cl_2$, and dried at room temperature. A total of 4 spectrophotometric ninhydrin colour tests indicated 1.00 mmol $NH_2$/g sheet (0.99, 0.96, 1.02, and 1.01 mmol/g, respectively), and elemental analysis indicated 1.07 mmol N/g sheet.

The following polystyrene-grafted polyethylene sheets have also been aminomethylated:

```
331% grafted sheet: substitution = 0.21 mmol NH₂/g sheet.
547% "       "       "      "      "   0.46 "    "      "
129% "       "       "      "      "   0.50 "    "      "
 46% "       "       "      "      "   0.02 "    "      "
285% "       "       "      "      "   0.6  "    "      "
```

EXAMPLE 7

*Preparation of BocGln – 4 – (oxymethyl) – Pam – sheet.*

0.63 g aminomethyl-sheet (substitution = 1.0 mmol/g sheet; 443% graft) was pre-washed in 30 ml DMF/$CH_2Cl_2$ (1:2 v/v) for 3 x 3 min in a 60 ml reaction vessel on a SPPS shaker. 0.98 g Boc-Gln-4-(oxymethyl)phenylacetic acid (2.5 mmol, 4 equiv.) and 0.38 g HOBt (2.5 mmol, 4 equiv.) were dissolved in 20 ml DMF/$CH_2Cl_2$ (1:1 v/v) and stirred in a screw-capped tube for 3 min at 0°C. 0.52 g DCC was dissolved in 10 ml $CH_2Cl_2$ and added to the mixture. After stirring for 25 min at 0°C, DCU was filtered off and the filtrate was added to the pre-washed aminomethyl-sheet and shaken for 2 h. The sheets were filtered, washed with $CH_2Cl_2$, neutralized with DIEA/$CH_2Cl_2$ (5:95 v/v), washed with $CH_2Cl_2$, and dried. The absence of positive ninhydrin tests indicated quantitative coupling, which was also confirmed after removal of the Boc group by the following treatment: 30 ml TFA/$CH_2Cl_2$ (1:1 v/v) for 1 x 2 min and 1 x 30 min, 30 ml $CH_2Cl_2$ for 6 x 1 min, 30 ml DIEA/$CH_2Cl_2$ (5:95 v/v) for 2 x 5 min, and 30 ml $CH_2Cl_2$ for 4 x 1 min. 2 ninhydrin tests then indicated the extent of -$NH_2$ substitution to be 0.76 mmol $NH_2$/g sheet (0.74 and 0.77 mmol/g, respectively), which is very close to the theoretical value of 0.78 mmol $NH_2$/g sheet.

15

EXAMPLE 8

*Peptide Synthesis: (a) Assembly of Protected Human [Asp[76]]−Parathyroid Hormone Fragments 80−84, 75−84 and 70−84 on 443 wt % Polystyrene−grafted Polyethylene Sheet.*

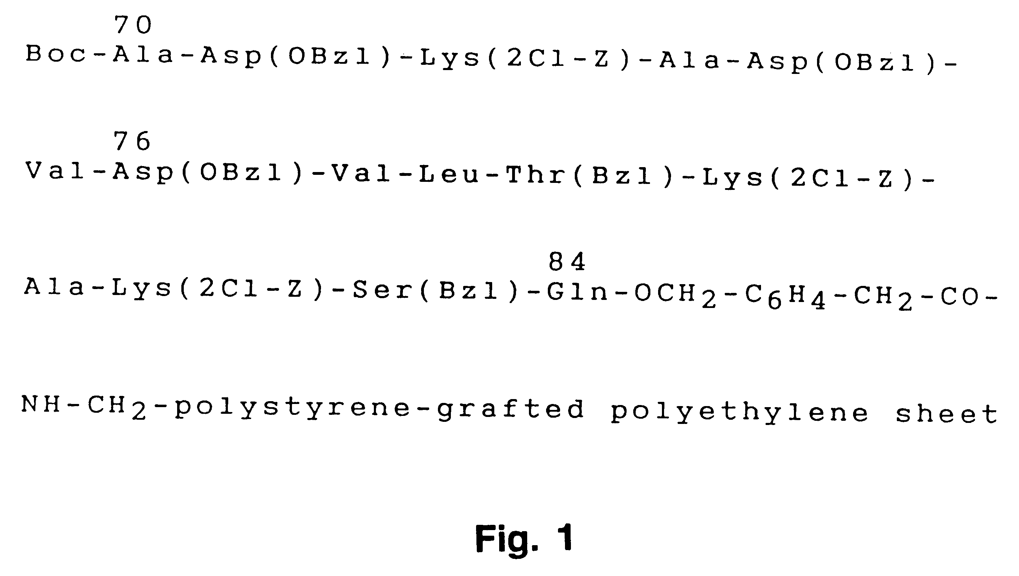

BocGln-OCH$_2$-Pam-sheet (0.80 g, 443% graft, 0.58 mmol Gln) was placed in a 60 ml reaction vessel on a SPPS shaker. Protected hPTH 70-84, (see Fig. 1) was assembled using the following synthetic protocol:

(1) CH$_2$Cl$_2$, 35 ml, 3x1 min;

(2) TFA/CH$_2$Cl$_2$ (1:1 v/v), 35 ml, 3x1 min;

(3) TFA/CH$_2$Cl$_2$ (1:1 v/v), 35 ml, 30 min;

(4) CH$_2$Cl$_2$, 35 ml, 6x1 min;

(5) DIEA/CH$_2$Cl$_2$ (1:19 v/v), 35 ml, 3x2 min;

(6) CH$_2$Cl$_2$, 35 ml, 6x1 min;

(7) 3 to 10 mg samples were cut off for ninhydrin analysis;

(8) protected amino acid was coupled as pre-formed symmetric anhydride (3 equiv., 0.05 M) in 35 ml DMF/CH$_2$Cl$_2$ (1:4 v/v), with shaking for 2 hours;

(9) CH$_2$Cl$_2$, 35 ml, 4x2 min;

(10) 3 to 10 mg samples were cut off and neutralized by repeating (5) and (6) before ninhydrin analysis.

All couplings were single couplings. Monitoring of the synthesis by using the quantitative ninhydrin test [originally developed for peptide synthesis on beads; see e.g. Sarin et al., Anal. Biochem., *117*, 147 (1981)] was successfully applied (Table 5), and with the exception (for unknown reasons) of the result for the second amino acid coupling (i.e. with formation of Boc-Ser[83] (Bzl)Gln[84]-OCH$_2$-Pam-sheet), indicated satisfactory values for the coupling efficiency in each coupling step.

TABLE 5

| Quantitative ninhydrin monitoring[a] of the solid-phase synthesis of protected human parathyroid hormone fragment (70-84) on 443 wt % polystyrene-grafted polyethylene | | | | |
|---|---|---|---|---|
| Residue coupled | Coupling[b] | | Deprotection | |
| | Remaining free amino groups ($\mu$mol/g) | Estimated[c] % completion | Measured substitution (mmol/g) | Theoretical substitution (mmol/g) |
| 84 BocGlnX[d] | 0.0 | 100 | 0.76 ± 0.02 | 0.78 |
| 83 BocSer(Bzl) | 38.0 | 94.0 | 0.35 ± 0.04 | 0.69 |
| 82 BocLys(2Cl-Z) | 1.6 | 99.7 | 0.54 ± 0.03 | 0.57 |
| 81 BocAla | 0.6 | 99.9 | 0.52 ± 0.03 | 0.55 |
| 80 BocLys(2Cl-Z) | 1.2 | 99.7 | 0.53 ± 0.02 | 0.47 |
| 79 BocThr(Bzl) | 0.0 | 100 | 0.44 ± 0.03 | 0.43 |
| 78 BocLeu | 0.4 | 99.9 | 0.39 ± 0.01 | 0.41 |
| 77 BocVal | 0.0 | 100 | 0.39 ± 0.03 | 0.40 |
| 76 BocAsp(OBzl) | 0.0 | 100 | 0.35 ± 0.01 | 0.37 |
| 75 BocVal | 0.2 | 99.9 | 0.31 ± 0.02 | 0.35 |
| 74 BocAsp(OBzl) | 0.7 | 99.8 | 0.31 ± 0.02 | 0.33 |
| 73 BocAla | 0.0 | 100 | 0.29 ± 0.01 | 0.33 |
| 72 BocLys(2Cl-Z) | 1.1 | 99.6 | 0.30 ± 0.02 | 0.30 |
| 71 BocAsp(OBzl) | 1.3 | 99.5 | 0.28 ± 0.02 | 0.28 |
| 70 BocAla | 0.0 | 100 | 0.23 ± 0.01 | 0.27 |

[a] Average values based on 2-4 ninhydrin analyses after coupling and deprotection in each cycle expressed as mmol/g of peptide-sheet.

[b] No residues were recoupled, but coupling of Boc-Ser(Bzl) was followed by complete acetylation of remaining free amino groups using N-acetylimidazole in methylene chloride.

[c] These estimated values are calculated relative to the theoretical substitution after coupling of the Boc-protected residue, and do not include correction for incomplete coupling of the preceding residue.

[d] X = -OCH$_2$-C$_6$H$_4$-CH$_2$-COOH.

*(b) Cleavage, purification and identification of synthetic hPTH−(80−84).*

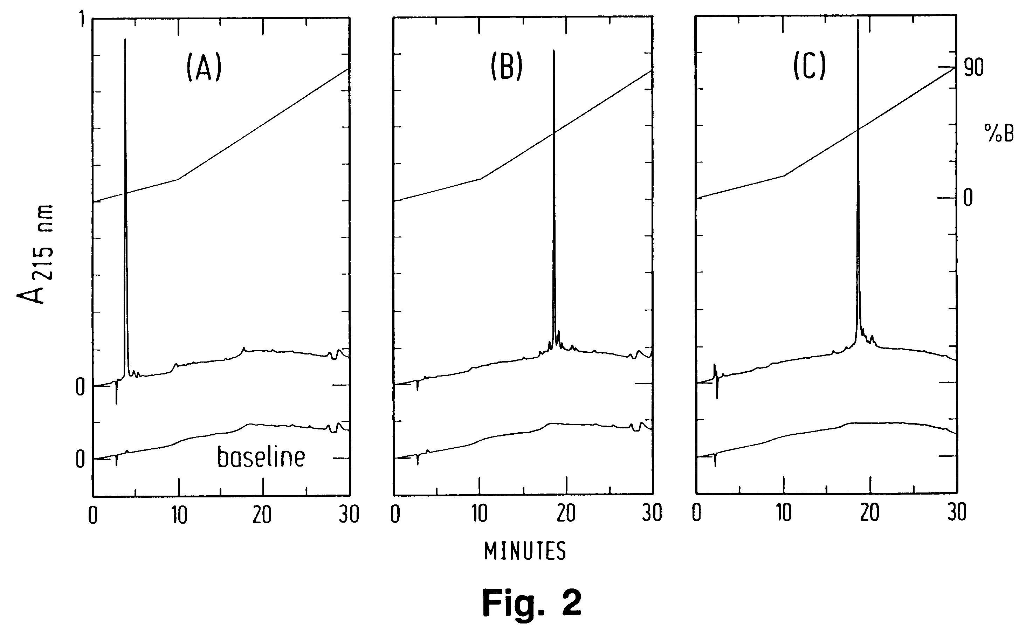

90 mg of H-Lys(ClZ)AlaLys(ClZ)Ser(Bzl)GlnOCH$_2$-Pam-sheet was treated with 5 ml of anhydrous HF/anisole (9:1 v/v) for 1 h at 0°C to simultaneously deprotect the side-chain protecting groups and cleave the peptide from the sheet. Extractions with ether, to remove organic components such as anisole and alkylated anisoles, were followed by extraction into 10% aqueous acetic acid. Lyophilization gave 24.0 mg of crude product of high purity [see HPLC chromatogram in Fig. 2 (A)].

The crude product was purified in two steps on a preparative C$_{18}$ column (300 x 19 mm). Buffers including TFA were evaporated off under reduced pressure and the product was redissolved in water; the solution was filtered and lyophilized to give 17.8 mg of H-LysAlaLysSerGln-OH, as confirmed by amino acid analysis (Table 6) and FABMS molecular weight measurements (Table 7).

The overall synthetic yield was approx. 84%, and the yield of pure peptide was approx. 69% based on the quantitative amino acid analysis.

*(c) Cleavage, purification and identification of synthetic hPTH−(75−84).*

93 mg of H-ValAsp(OBzl)ValLeuThr(Bzl)Lys(ClZ)AlaLys(ClZ)Ser(Bzl)Gln-OCH$_2$-Pam-sheet was treated in the same manner as for hPTH-(80-84), giving 36.6 mg of crude product [see HPLC chromatogram in Fig. 2 (B)], and finally 27.2 mg of purified peptide, identified by amino acid analysis (Table 6) and molecular weight measurements (Table 7). The overall synthetic yield was approx. 85%, and the yield of pure peptide was approx. 69%, based on the quantitative amino acid analysis.

*(d) Cleavage, purification and identification of synthetic hPTH−(70−84).*

The hPTH-(70-84) fragment was released from 96 mg of peptide-sheet in the same way as in *(b)* and *(c)*, above [see HPLC chromatogram in Fig. 2 (C)]. The overall synthetic yield was approx. 83% and the yield of pure peptide was 26 mg (approx. 63%). The pure peptide was identified by amino acid analysis (Table 6) and molecular weight measurements (Table 7).

TABLE 6

| Amino acid composition of purified synthetic hPTH compounds | | | |
|---|---|---|---|
| Amino acid | Molar ratio[a] | | |
| | hPTH-(70-84) | hPTH-(75-84) | hPTH-(80-84) |
| Asp | 2.94 (3) | 1.01 (1) | - |
| Thr[b] | 0.99 (1) | 0.95 (1) | - |
| Ser[b] | 1.05 (1) | 0.81 (1) | 0.94 (1) |
| Glu | 1.31 (1) | 1.03 (1) | 1.07 (1) |
| Ala | 3.00 (3) | 1.00 (1) | 1.00 (1) |
| Val | 2.00 (2) | 2.00 (2) | - |
| Leu | 1.03 (1) | 1.00 (1) | - |
| Lys | 3.08 (3) | 1.98 (2) | 1.89 (2) |

[a] Values in parentheses are theoretical.
[b] Thr and Ser values were not corrected for loss during hydrolysis.

Hydrolyses were performed in sealed, evacuated tubes with 5.7 M HCl containing 0.05 % phenol, 110°C, 20 h. Analysis by HPLC using fluorescence detection (338/450 nm) following treatment (post-column) with an *o*-phthaldialdehyde derivatizing reagent.

TABLE 7

| Molecular weights of hPTH compounds | | |
|---|---|---|
| Peptide | Molecular weight | |
| | measured | calculated |
| hPTH-(80-84) | 560.3 | 560.3 |
| hPTH-(75-84) | 1088 | 1088 |
| hPTH-(70-84) | 1588 | 1588 |

Determined by quadrupole mass spectrometry. The calculated m/z values are for C = 12.000 u and H = 1.008 u.

EXAMPLE 9

*Rapid parallel synthesis of multiple peptide analogs:*

**(a) Labeling of sheets**

Aminomethylated 285 wt % polystyrene-grafted polyethylene sheet (0.6 mmol $NH_2$/g sheet) was cut into thirteen discrete pieces (each piece: 1.5 x 3 cm, ca. 50 $\mu$m thickness, ca. 40 mg) and labeled individually by means of graphite-based ink. A piece of polyethylene film was placed on top of each labeled surface and melted into the grafted sheet by using an electrically heated sealing apparatus. Finally, the sheets were shaken in 50% TFA/$CH_2Cl_2$ for 20 min. to check that all labels were adequately sealed in.

**(b) Simultaneous synthesis of melittin-(7-21) and twelve analogs on labeled sheets**

Protected melittin-(7-21), i.e.

```
        7                                    12        14
   Boc-Lys(ClZ)-Val-Leu-Thr(Bzl)-Thr(Bzl)-Gly-Leu-Pro-Ala-Leu-Ile-
                 21
   Ser(Bzl)-Trp(CHO)-Ile-Lys(ClZ)-Pam-sheet,
```

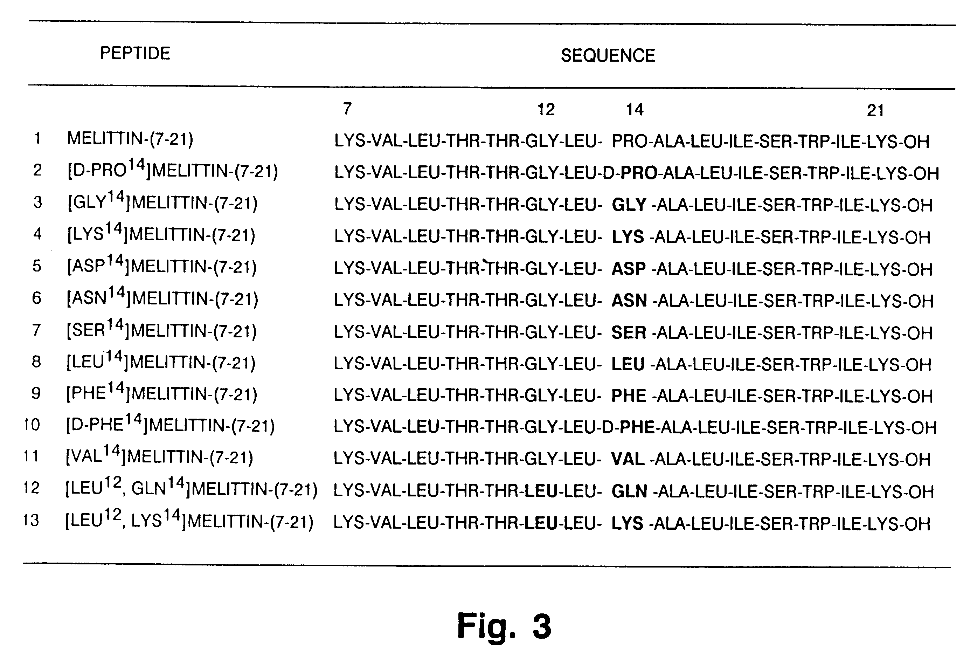

and twelve analogs derived by substitutions in positions 12 and 14 (sequences of the free peptides are listed in Fig. 3) were each assembled stepwise on a labeled sheet. The common steps of deprotection, neutralization, washing and coupling of identical amino acids were performed simultaneously in a single reaction vessel, while the coupling of different amino acids was carried out in separate vessels.

A standard solid-phase procedure was employed, using double DCC coupling (3.5 equiv., 0.05 M, in 30% DMF/$CH_2Cl_2$) of all residues except for Boc-Gln and Boc-Asn, which were double coupled as HOBt esters in 30% DMF/$CH_2Cl_2$, and Boc-Leu[13] to Gln[14], which was double coupled as a symmetric anhydride in 20% DMF/$CH_2Cl_2$.

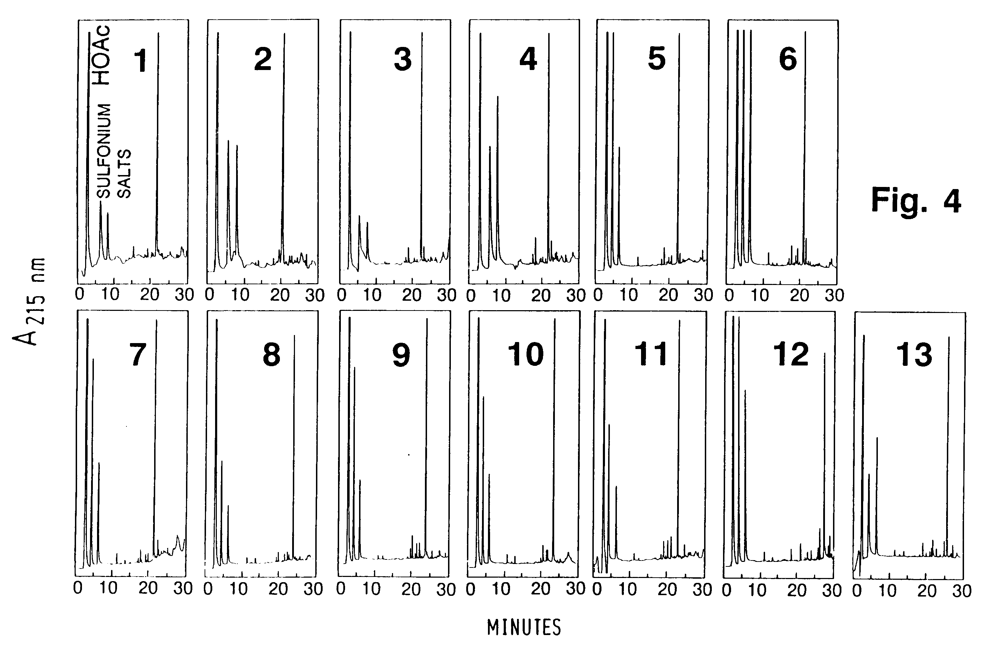

Following removal of the N-terminal Boc group, deprotection and release of the peptides from the sheets were accomplished by the low/high HF method (Tam et al., *J. Am. Chem. Soc.*, 105, 6442 (1983)). The free 15-residue peptides were obtained in overall synthetic yields of ca. 70%. Fig. 4 shows HPLC chromatograms of the thirteen unpurified peptides. All peptides were purified in 1-2 steps on a semi-preparative $C_{18}$ column. As an example, 3.2 mg of pure melittin-(7-21) was obtained from 1 cm$^2$ (23.2 mg) of fully protected peptide-sheet. The identity of the peptides was verified by amino acid analysis (Table 8) and molecular weight measurements (Table 9).

TABLE 8

Amino acid composition of purified melittin-(7-21) and its analogs

| Amino acid | Molar ratio[a] | | | | |
| --- | --- | --- | --- | --- | --- |
| | pept. 1 | pept. 2 | pept. 3 | pept. 4 | pept. 5 |
| Lys | 1.95 (2) | 1.69 (2) | 1.77 (2) | 2.83 (3) | 1.47 (2) |
| Val | 0.65 (1) | 0.82 (1) | 0.87 (1) | 0.78 (1) | 0.87 (1) |
| Leu | 2.97 (3) | 2.85 (3) | 2.94 (3) | 2.67 (3) | 2.86 (3) |
| Thr[b] | 1.87 (2) | 1.89 (2) | 1.93 (2) | 1.70 (2) | 1.84 (2) |
| Gly | 1.08 (1) | 1.03 (1) | 2.04 (2) | 1.07 (1) | 1.12 (1) |
| Pro | 0.98 (1) | 0.98 (1) | - | - | - |
| Ala | 1.00 (1) | 1.00 (1) | 1.00 (1) | 1.00 (1) | 1.00 (1) |
| Ile | 1.93 (2) | 1.75 (2) | 1.81 (2) | 1.75 (2) | 1.77 (2) |
| Ser[b] | 0.90 (1) | 0.96 (1) | 0.97 (1) | 1.00 (1) | 0.98 (1) |
| Trp[c] | | | | | |
| Asp | - | - | - | - | (1) |

| | pept. 6 | pept. 7 | pept. 8 | pept. 9 | pept. 10[d] |
| --- | --- | --- | --- | --- | --- |
| Lys | 1.87 (2) | 1.71 (2) | 1.83 (2) | 1.93 (3) | (2) |
| Val | 0.88 (1) | 0.81 (1) | 0.88 (1) | 0.87 (1) | (1) |
| Leu | 2.92 (3) | 2.93 (3) | 3.90 (4) | 3.06 (3) | (3) |
| Thr[b] | 1.87 (2) | 1.90 (2) | 1.88 (2) | 2.06 (2) | (2) |
| Gly | 1.09 (1) | 1.08 (1) | 1.05 (1) | 1.26 (1) | (1) |
| Ala | 1.00 (1) | 1.00 (1) | 1.00 (1) | 1.00 (1) | (1) |
| Ile | 1.82 (2) | 1.80 (2) | 1.83 (2) | 1.95 (2) | (2) |
| Ser[b] | 0.95 (1) | 1.88 (2) | 0.95 (1) | 1.01 (1) | (1) |
| Trp[c] | | | | | |
| Asp | 1.04 (1) | - | - | - | |
| Phe | - | - | - | 0.91 (1) | (1) |

19

|         | pept. 11    | pept. 12    | pept. 13    |
| ------- | ----------- | ----------- | ----------- |
| Lys     | 1.75 (2)    | 1.66 (2)    | 2.62 (3)    |
| Val     | 1.75 (2)    | 0.81 (1)    | 0.89 (1)    |
| Leu     | 2.73 (3)    | 3.70 (4)    | 3.71 (4)    |
| Thr[b]  | 1.92 (2)    | 1.62 (2)    | 1.93 (2)    |
| Gly     | 1.05 (1)    | -           | -           |
| Ala     | 1.00 (1)    | 1.00 (1)    | 1.00 (1)    |
| Ile     | 1.73 (2)    | 1.73 (2)    | 1.80 (2)    |
| Ser[b]  | 0.97 (1)    | 1.00 (1)    | 0.95 (1)    |
| Trp[c]  |             |             |             |
| Glu     | -           | 1.12 (1)    | -           |

The peptides were hydrolyzed in 5.7 M HCl at 110°C for 18 h in sealed non-evacuated tubes, except peptide 1 which was hydrolyzed for 24 h in an evacuated tube. After filtration, hydrolysates were analyzed on a Beckman 6300 amino acid analyzer.

[a] Values in parentheses are theoretical.

[b] Thr and Ser values were not corrected for loss during hydrolysis.

[c] Tryptophan was not determined.

[d] Peptide analog number 10 was not analyzed.

TABLE 9

| Molecular weights of melittin-(7-21) analogs[a] | | | |
|---|---|---|---|
| Peptide | Molecular weight | | |
| | measured | calculated | $\triangle$[b] |
| 1 | 1640.5 | 1640.0 | + 0.5 |
| 2 | 1640.1 | 1640.0 | + 0.1 |
| 3 | 1600.0 | 1599.9 | + 0.1 |
| 4 | 1671.2 | 1671.1 | + 0.1 |
| 5 | 1658.0 | 1658.0 | 0.0 |
| 6 | 1657.0 | 1657.0 | 0.0 |
| 7 | 1630.2 | 1630.0 | + 0.2 |
| 8 | 1656.1 | 1656.0 | + 0.1 |
| 9 | 1690.0 | 1690.1 | -0.1 |
| 10 | 1690.3 | 1690.1 | + 0.2 |
| 11 | 1642.2 | 1642.0 | + 0.2 |
| 12 | 1727.4 | 1727.1 | + 0.3 |
| 13 | 1727.3 | 1727.2 | + 0.1 |

[a] Determined by $^{252}$Cf fission fragment time of flight mass spectrometry from the mean of the most abundant isotopes.
[b] $\triangle$ = measured - calculated.

**Claims**

1. A method for the synthesis of a peptide or a protein, the method comprising the steps of:

A) providing a polymer substrate grafted with polystyrene chains, said polystyrene chains optionally further bearing substituents which are not reactive under the conditions prevailing in the synthesis, the estimated peak molecular weight of the polystyrene chains grafted to the polymer, not including optional substituents, being at least 200,000, at least part of the polystyrene chains of the polystyrene-grafted polymer substrate being functionalized with a chemical functionality facilitating the formation of an anchoring linkage between the polystyrene moiety and an at least N-protected and optionally carboxyl terminal derivatized amino acid,

B) coupling an N-protected and optionally carboxyl terminal derivatized amino acid to the functionalized polystyrene moiety, said functionality and said N-protected and optionally carboxyl terminal derivatized amino acid being adapted to each other such that the anchoring linkage formed can subsequently be cleaved substantially without degradation of the peptide or protein chain which is to be synthesized,

C) removing the N-protecting group from an N-protected amino or substituted amino group of the coupled and N-protected amino acid, such that reaction of the amino or substituted amino group of the coupled amino acid with a carboxyl group or an activated carboxyl group of a further amino acid is facilitated,

D) reacting said amino or substituted amino group of the last-coupled amino acid with a carboxyl group or an activated carboxyl group of a further N-protected amino acid so as to form a peptide bond between the two amino acid moieties,

E) optionally removing the N-protecting group from an N-protected amino or substituted amino group of the last-coupled N-protected amino acid, such that reaction of the amino or substituted amino group of the latter amino acid with a carboxyl group or activated carboxyl group of a further N-protected amino acid is facilitated,

F) in those cases where step E) has been performed, repeating steps D) and E) a desired number of times,

G) optionally removing some or all protecting groups possibly remaining on the amino acid moieties of the synthesized peptide or protein chain,

H) optionally cleaving the linkage anchoring the synthesized peptide or protein chain to the functionalized polystyrene moiety,

21

and

    l) optionally removing any further undesired group from the synthesized peptide or protein chain.

**2.** A method for the synthesis of one or more peptides or proteins, which method, when two or more peptides or proteins are to be synthesized, permits the parallel and substantially simultaneous synthesis of the desired number of peptides and proteins, the method comprising:

    A) providing a plurality of substantially identical polymer substrates grafted with polystyrene chains, said polystyrene chains optionally further bearing substituents which are not reactive under the conditions prevailing in the synthesis, the estimated peak molecular weight of the polystyrene chains grafted to the polymer, not including optional substituents, being at least 200,000, at least part of the polystyrene chains of each polystyrene-grafted polymer substrate being functionalized with a chemical functionality facilitating the formation of an anchoring linkage between the polystyrene moieties and an at least *N*-protected and optionally carboxyl terminal derivatized amino acid,

    B) optionally physically segregating the members of said plurality of polystyrene-grafted polymer substrates into two or more sets each comprising one or more members of said plurality, coupling an *N*-protected and optionally carboxyl terminal derivatized amino acid to the functionalized polystyrene moieties of each member of said plurality or, where applicable, each member of each set, the *N*-protected and optionally carboxyl terminal derivatized amino acid employed being identical for all the members of the plurality or, where applicable, all the members of one set, and, where applicable, further being in accordance with one of the following alternatives:

        (i) identical for all the sets,

        (ii) when the number of said sets is greater than two, identical for at least two of the sets,

        (iii) different for each set,

said functionality and said *N*-protected and optionally carboxyl terminal derivatized amino acid being adapted to each other such that the anchoring linkage formed can subsequently be cleaved substantially without degradation of the peptide or protein chain which is to be synthesized,

    C) treating each member of said plurality or, where applicable, each member of each said set so as to remove the *N*-protecting group from an *N*-protected amino or substituted amino group of the coupled and *N*-protected amino acid, such that reaction of the amino or substituted amino group of the coupled amino acid with a carboxyl group or an activated carboxyl group of a further *N*-protected amino acid is facilitated,

    D) reacting said amino or substituted amino group of the amino acid last coupled to the functionalized polystyrene moieties of each member of said plurality or, where applicable, of each member of each set with a carboxyl group or an activated carboxyl group of a further *N*-protected amino acid, so as to form a peptide bond between said amino or substituted amino group and said carboxyl group or activated carboxyl group, said further *N*-protected amino acid being identical for all the members of the plurality or, where applicable, all the members of one set, and, where applicable, further being in accordance with one of the three alternatives mentioned above in connection with step B),

    E) optionally treating each member of said plurality or, where applicable, each member of each said set so as to remove the *N*-protecting group from an *N*-protected amino or substituted amino group of the last-coupled *N*-protected amino acid, such that reaction of the amino or substituted amino group of the latter amino acid with a carboxyl group or activated carboxyl group of a further *N*-protected amino acid is facilitated,

    F) in those cases where step E) has been performed, repeating steps D) and E) a desired number of times,

    G) optionally treating each member of said plurality or, where applicable, each member of each said set so as to remove some or all protecting groups possibly remaining on the amino acid moieties of the synthesized peptide or protein chain,

    H) optionally treating each member of said plurality or, where applicable, each member of each said set so as to cleave the linkage anchoring the synthesized peptide or protein chain to the functionalized polystyrene moieties of each member of said plurality or, where applicable, of each member of each said set,

    and

    l) optionally removing any further undesired group from a synthesized peptide or protein chain.

**3.** A method for the preparation of a polystyrene-grafted polymer substrate bearing a peptide or protein which has been synthesized thereon, the method comprising the steps of:

A) providing a polymer substrate grafted with polystyrene chains, said polystyrene chains optionally further bearing substituents which are not reactive under the conditions prevailing in the synthesis, the estimated peak molecular weight of the polystyrene chains grafted to the polymer, not including optional substituents, being at least 200,000, at least part of the polystyrene chains of the polystyrene-grafted polymer substrate being functionalized with a chemical functionality facilitating the formation of an anchoring linkage between the polystyrene moiety and an at least *N*-protected and optionally carboxyl terminal derivatized amino acid,

B) coupling an *N*-protected and optionally carboxyl terminal derivatized amino acid to the functionalized polystyrene moiety, said functionality and said *N*-protected and optionally carboxyl terminal derivatized amino acid being adapted to each other such that the anchoring linkage formed can subsequently be cleaved substantially without degradation of the peptide or protein chain which is to be synthesized,

C) removing the *N*-protecting group from an *N*-protected amino or substituted amino group of the coupled and *N*-protected amino acid, such that reaction of the amino or substituted amino group of the coupled amino acid with a carboxyl group or an activated carboxyl group of a further amino acid is facilitated,

D) reacting said amino or substituted amino group of the last-coupled amino acid with a carboxyl group or an activated carboxyl group of a further *N*-protected amino acid so as to form a peptide bond between the two amino acid moieties,

E) optionally removing the *N*-protecting group from an *N*-protected amino or substituted amino group of the last-coupled *N*-protected amino acid, such that reaction of the amino or substituted amino group of the latter amino acid with a carboxyl group or activated carboxyl group of a further *N*-protected amino acid is facilitated,

F) in those cases where step E) has been performed, repeating steps D) and E) a desired number of times,

and

G) optionally removing some or all protecting groups possibly remaining on the amino acid moieties of the synthesized peptide or protein chain,

4. A method as claimed in any one of claims 1 to 3, wherein a said polystyrene-grafted polymer substrate has been formed by a substantially radical-initiated reaction between the polymer substrate and optionally substituted styrene monomer present in a solution of said monomer in an organic solvent.

5. A method as claimed in claim 4, wherein the organic solvent which has been used to dissolve the optionally substituted styrene monomer is an alcohol.

6. A method as claimed in claim 5, wherein said alcohol is methanol.

7. A method as claimed in claim 6, wherein the volume percentage (% v/v) of optionally substituted styrene in the solution which has been used is such that $25 \leq \% \text{ v/v} \leq 35$.

8. A method as claimed in any one of the preceding claims, wherein the estimated peak molecular weight of the polystyrene chains grafted to the polymer, not including optional substituents, is in the range of 300,000-1,600,000.

9. A method as claimed in any one of the preceding claims, wherein the polystyrene-grafted polymer substrate has been prepared from a polymer substrate in the form of a sheet or film of thickness in the range of 25 to 100 $\mu$m, and the degree of polystyrene chain grafting of the polymer substrate is in the range of 5-800% by weight.

10. A method as claimed in claim 9, wherein the degree of polystyrene chain grafting of the polymer substrate is in the range of 100-600% by weight.

11. A method as claimed in any one of the preceding claims, wherein the polystyrene-grafted polymer substrate is in the form of a sheet or film.

12. A method as claimed in any one of claims 1 to 8, wherein the polystyrene-grafted polymer substrate has been prepared from a polymer substrate in the form of a bead, pellet, disc, ring, tube, rod or net.

23

**13.** A method as claimed in any one of the preceding claims, wherein the chemical functionality facilitating the formation of an anchoring linkage between an at least *N*-protected and optionally derivatized amino acid and the functionalized polystyrene moiety is, or is derived from,

a chloro-, bromo- or iodo-substituted alkyl,

an amino-substituted alkyl,

an amino- and aryl-substituted alkyl,

an amino- and alkylaryl-substituted alkyl, or

an hydroxy-substituted alkyl,

the functionality, if derived from any hereof, being a functionality with a spacer group such that a synthesized peptide or protein chain will be cleavable from the polystyrene moiety substantially without degradation of said chain.

**14.** A method as claimed in claim 13, wherein said chemical functionality facilitating the formation of an anchoring linkage is, or is derived from,

chloromethyl,

aminomethyl,

$\alpha$-aminobenzyl,

$\alpha$-amino-2-, $\alpha$-amino-3- or $\alpha$-amino-4-methylbenzyl, or

hydroxymethyl.

**15.** A method as claimed in any one of the preceding claims, wherein the polymer is a polyolefin.

**16.** A method as claimed in claim 15, wherein said polyolefin is polyethylene.

**17.** A peptide- or protein-bearing polystyrene-grafted polymer substrate prepared by a method as claimed in any one of claims 3 to 16.

**18.** A polymer substrate grafted with polystyrene chains and to which a peptide or protein is coupled, said polystyrene chains optionally further bearing substituents which are not reactive under the conditions prevailing in peptide synthesis, the estimated peak molecular weight of the polystyrene chains grafted to the polymer, not including optional substituents, being at least 200,000, at least part of the polystyrene chains of the polystyrene-grafted polymer substrate bearing an anchoring linkage via which said peptide or protein is coupled.

**19.** A polystyrene-grafted polymer substrate as claimed in claim 18, the estimated peak molecular weight of the polystyrene chains grafted to the polymer, not including optional substituents, being in the range of 300,000-1,600,000.

**20.** A polystyrene-grafted polymer substrate as claimed in claim 18 or 19, which has been prepared from a polymer substrate in the form of a sheet or film of thickness in the range of 25 to 100 $\mu$m, and in which the degree of polystyrene chain grafting of the polymer substrate is in the range of 5-800% by weight.

**21.** A polystyrene-grafted polymer substrate as claimed in claim 20, wherein the degree of polystyrene chain grafting of the polymer substrate is in the range of 100-600% by weight.

**22.** A polystyrene-grafted polymer substrate as claimed in any one of claims 18 to 21, which is in the form of a sheet or film.

**23.** A polystyrene-grafted polymer substrate as claimed in claim 18 or 19, which has been prepared from a polymer substrate in the form of a bead, pellet, disc, ring, tube, rod or net.

**24.** A polystyrene-grafted polymer substrate as claimed in any one of claims 18 to 23, wherein the polymer is a polyolefin.

**25.** A polystyrene-grafted polymer substrate as claimed in claim 24, wherein said polyolefin is polyethylene.

24

26. A polymer substrate grafted with polystyrene chains, said polystyrene chains optionally further bearing substituents which are not reactive under the conditions prevailing in peptide synthesis, the estimated peak molecular weight of the polystyrene chains grafted to the polymer, not including optional substituents, being at least 200,000, at least part of the polystyrene chains of the polystyrene-grafted polymer substrate being functionalized with a chemical functionality facilitating the formation of an anchoring linkage between the polystyrene moiety and an at least *N*-protected and optionally carboxyl terminal derivatized amino acid.

27. A polystyrene-grafted polymer substrate as claimed in claim 26, wherein the estimated peak molecular weight of the polystyrene chains grafted to the polymer, not including optional substituents, is in the range of 300,000-1,600,000.

28. A polystyrene-grafted polymer substrate as claimed in claim 26 or 27, which has been prepared from a polymer substrate in the form of a sheet or film of thickness in the range of 25 to 100 $\mu$m, and in which the degree of polystyrene chain grafting of the polymer substrate is in the range of 5-800% by weight.

29. A polystyrene-grafted polymer substrate as claimed in claim 28, wherein the degree of polystyrene chain grafting of the polymer substrate is in the range of 100-600% by weight.

30. A polystyrene-grafted polymer substrate as claimed in any one of claims 26 to 29, which is in the form of a sheet or film.

31. A polystyrene-grafted polymer substrate as claimed in claim 26 or 27, which has been prepared from a polymer substrate in the form of a bead, pellet, disc, ring, tube, rod or net.

32. A polystyrene-grafted polymer substrate as claimed in any one of claims 26 to 31, wherein the chemical functionality facilitating the formation of an anchoring linkage between an at least *N*-protected and optionally derivatized amino acid and the functionalized polystyrene moiety is, or is derived from,
a chloro-, bromo- or iodo-substituted alkyl,
an amino-substituted alkyl,
an amino- and aryl-substituted alkyl,
an amino- and alkylaryl-substituted alkyl, or
an hydroxy-substituted alkyl,
the functionality, if derived from any hereof, being a functionality with a spacer group such that a synthesized peptide or protein chain will be cleavable from the polystyrene moiety substantially without degradation of said chain.

33. A polystyrene-grafted polymer substrate as claimed in claim 32, wherein said chemical functionality facilitating the formation of an anchoring linkage is, or is derived from,
chloromethyl,
aminomethyl,
$\alpha$-aminobenzyl,
$\alpha$-amino-2-, $\alpha$-amino-3- or $\alpha$-amino-4-methylbenzyl, or
hydroxymethyl.

34. A polystyrene-grafted polymer substrate as claimed in claim 32 or 33, wherein the functionality is derived from an amino group-containing moiety selected from: amino-substituted alkyl; amino- and aryl-substituted alkyl; and amino- and alkylaryl-substituted alkyl; and the functionality comprises a spacer group derived from a 4-(haloalkyl)aryl-lower alkanoic acid, a Boc-aminoacyl-4-(oxymethyl)-aryl-lower alkanoic acid, an *N*-Boc-*p*-acylbenzhydrylamine, an *N*-Boc-4'-lower alkyl-*p*-acylbenzhydrylamine, an *N*-Boc-4'-lower alkoxy-*p*-acylbenzhydrylamine or a 4-hydroxymethylphenoxy-lower alkanoic acid.

35. A polystyrene-grafted polymer substrate as claimed in any one of claims 26 to 34, wherein the polymer is a polyolefin.

36. A polystyrene-grafted polymer substrate as claimed in claim 35, wherein said polyolefin is polyethylene.

**37.** A method for the preparation of a functionalized polystyrene-grafted polymer substrate as claimed in any one of claims 26 to 36 comprising subjecting a polymer substrate immersed in a solution of optionally substituted styrene monomer in an organic solvent to a treatment leading to the formation of free radicals such that polystyrene chains are grafted to the polymer substrate, and functionalizing at least part of the polystyrene chains with a chemical functionality facilitating the formation of an anchoring linkage between the polystyrene moiety and an at least N-protected and optionally carboxyl terminal derivatized amino acid.

**38.** A method for the preparation of a polymer substrate grafted with polystyrene chains and to which an at least N-protected amino acid is coupled, said polystyrene chains optionally further bearing substituents which are not reactive under the conditions prevailing in peptide synthesis, the estimated peak molecular weight of the polystyrene chains grafted to the polymer, not including optional substituents, being at least 200,000, at least part of the polystyrene chains of the polystyrene-grafted polymer substrate bearing an anchoring linkage via which said at least N-protected amino acid is coupled, the method comprising subjecting a polymer substrate immersed in a solution of optionally substituted styrene monomer in an organic solvent to a treatment leading to the formation of free radicals such that polystyrene chains are grafted to the polymer substrate, functionalizing at least part of the polystyrene chains with a chemical functionality facilitating the formation of an anchoring linkage between the polystyrene moiety and an at least N-protected and optionally carboxyl terminal derivatized amino acid, and reacting said functionality so as to form an anchoring linkage to an at least N-protected amino acid.

**39.** A method as claimed in claim 37 or 38, wherein the treatment leading to the formation of free radicals is gamma irradiation.

**40.** A method as claimed in claim 39, wherein the gamma irradiation is performed using a gamma radiation dose rate of from about 300 to about 1,000 Gy/hour.

**41.** A method as claimed in any one of claims 37 to 40, wherein the organic solvent is an alcohol.

**42.** A method according to claim 41, wherein said alcohol is methanol.

**43.** A method as claimed in any one of claims 37 to 42, wherein the volume percentage (% v/v) of optionally substituted styrene in the solution is such that $25 \leq \% \ v/v \leq 35$.

**44.** A method as claimed in any one of claims 37 to 43, wherein the grafting is performed so as to give an estimated peak molecular weight of the polystyrene chains grafted to the polymer, not including optional substituents, in the range of 300,000-1,600,000.

**45.** A method as claimed in any one of claims 37 to 44, wherein the polymer substrate is in the form of a sheet or film of thickness in the range of 25 to 100 $\mu$m, and the grafting is performed so as to give a degree of polystyrene chain grafting of the polymer substrate in the range of 5-800% by weight.

**46.** A method according to claim 45, wherein the the grafting is performed so as to give a degree of polystyrene chain grafting of the polymer substrate in the range of 100-600% by weight.

**47.** A method as claimed in any one of claims 37 to 44, wherein the polymer substrate is in the form of a sheet or film.

**48.** A method as claimed in claim 47, wherein the polymer substrate has a thickness of from 25 to 100 $\mu$m.

**49.** A method as claimed in any one of claims 37 to 44, wherein the polymer substrate is in the form of a bead, pellet, disc, ring, tube, rod or net.

**50.** A method as claimed in any one of claims 37 to 49, wherein the chemical functionality facilitating the formation of an anchoring linkage between an at least N-protected and optionally derivatized amino acid and the functionalized polystyrene moiety is, or is derived from,
a chloro-, bromo- or iodo-substituted alkyl,
an amino-substituted alkyl,

an amino- and aryl-substituted alkyl,

an amino- and alkylaryl-substituted alkyl, or

an hydroxy-substituted alkyl,

the functionality, if derived from any hereof, being a functionality with a spacer group such that a synthesized peptide or protein chain will be cleavable from the polystyrene moiety substantially without degradation of said chain.

51. A method as claimed in claim 50, wherein said chemical functionality facilitating the formation of an anchoring linkage is, or is derived from,

chloromethyl,

aminomethyl,

$\alpha$-aminobenzyl,

$\alpha$-amino-2-, $\alpha$-amino-3- or $\alpha$-amino-4-methylbenzyl, or

hydroxymethyl.

52. A method as claimed in claim 37 or 38, wherein the functionality is derived from an amino group-containing moiety selected from: amino-substituted alkyl; amino- and aryl-substituted alkyl; and amino- and alkylaryl-substituted alkyl; and the functionality is treated to provide the functionality with a spacer group derived from a 4-(haloalkyl)aryl-lower alkanoic acid, a Boc-aminoacyl-4-(oxymethyl)aryl-lower alkanoic acid, an $N$-Boc-$p$-acylbenzhydrylamine, an $N$-Boc-4'-lower alkyl-$p$-acylbenzhydrylamine, an $N$-Boc-4'-lower alkoxy-$p$-acylbenzhydrylamine or a 4-hydroxymethylphenoxy-lower alkanoic acid.

53. A method as claimed in any one of claims 37 to 52, wherein the polymer is a polyolefin.

54. A method as claimed in claim 53, wherein said polyolefin is polyethylene.

55. A polymer substrate grafted with polystyrene chains and to which an at least $N$-protected amino acid is coupled, said polystyrene chains optionally further bearing substituents which are not reactive under the conditions prevailing in peptide synthesis, the estimated peak molecular weight of the polystyrene chains grafted to the polymer, not including optional substituents, being at least 200,000, at least part of the polystyrene chains of the polystyrene-grafted polymer substrate bearing an anchoring linkage via which said at least $N$-protected amino acid is coupled.

56. A polystyrene-grafted polymer substrate as claimed in claim 55, the estimated peak molecular weight of the polystyrene chains grafted to the polymer, not including optional substituents, being in the range of 300,000-1,600,000.

57. A polystyrene-grafted polymer substrate as claimed in claim 55 or 56, which has been prepared from a polymer substrate in the form of a sheet or film of thickness in the range of 25 to 100 $\mu$m, and in which the degree of polystyrene chain grafting of the polymer substrate is in the range of 5-800% by weight.

58. A polystyrene-grafted polymer substrate as claimed in claim 57, wherein the degree of polystyrene chain grafting of the polymer substrate is in the range of 100-600% by weight.

59. A polystyrene-grafted polymer substrate as claimed in any one of claims 55 to 58, which is in the form of a sheet or film.

60. A polystyrene-grafted polymer substrate as claimed in claim 55 or 56, which has been prepared from a polymer substrate in the form of a bead, pellet, disc, ring, tube, rod or net.

61. A polystyrene-grafted polymer substrate as claimed in any one of claims 55 to 60, wherein the polymer is a polyolefin.

62. A polystyrene-grafted polymer substrate as claimed in claim 61, wherein said polyolefin is polyethylene.

63. A polymer substrate grafted with polystyrene chains, said polystyrene chains optionally further bearing substituents, the estimated peak molecular weight of the polystyrene chains grafted to the polymer, not including optional substituents, being at least 200,000.

64. A polystyrene-grafted polymer substrate as claimed in claim 63, wherein the estimated peak molecular weight of the polystyrene chains grafted to the polymer, not including optional substituents, is in the range of 300,000-1,600,000.

65. A polystyrene-grafted polymer substrate as claimed in claim 63 or 64, which has been prepared from a polymer substrate in the form of a sheet or film of thickness in the range of 25 to 100 $\mu$m, and in which the degree of polystyrene chain grafting of the polymer substrate is in the range of 5-800% by weight.

66. A polystyrene-grafted polymer substrate as claimed in claim 65, wherein the degree of polystyrene chain grafting of the polymer substrate is in the range of 100-600% by weight.

67. A polystyrene-grafted polymer substrate as claimed in any one of claims 63 to 66, which is in the form of a sheet or film.

68. A polystyrene-grafted polymer substrate as claimed in claim 63 or 64, which has been prepared from a polymer substrate in the form of a bead, pellet, disc, ring, tube, rod or net.

69. A polystyrene-grafted polymer substrate as claimed in any one of claims 63 to 68, wherein the polymer is a polyolefin.

70. A polystyrene-grafted polymer substrate as claimed in claim 69, wherein said polyolefin is polyethylene.

71. The use, in an analytical procedure, of a peptide-bearing polystyrene-grafted polymer substrate as claimed in any one of claims 17 to 25 as a light-transparent carrier material facilitating the spectrophotometric monitoring of an antigen/antibody reaction, said antigen being a peptide synthesized on and remaining anchored to the polystyrene-grafted polymer substrate.

72. The use as claimed in claim 71, said monitoring entailing the use of an ELISA technique.

**Patentansprüche**

1. Verfahren zur Synthese eines Peptids oder eines Proteins, wobei das Verfahren die Schritte umfaßt:
   A) Bereitstellen eines Polymersubstrats, das mit Polystyrolketten gepropft ist, wobei die Polystyrolketten gegebenenfalls weiter Substituenten tragen, die unter den in der Synthese vorherrschenden Bedingungen nicht reagieren, wobei das geschätzte Peak-Molekulargewicht der auf das Polymer gepropften Polystyrolketten ohne Einschluß gegebenenfalls vorhandener Substituenten mindestens 200.000 ist, wobei mindestens ein Teil der Polystyrolketten des mit Polystyrol gepropften Polymersubstrates mit einer chemischen Funktionalität funktionalisiert sind, welche die Bildung einer verankernden Bindung zwischen dem Polystyrolbestandteil und einer mindestens N-geschützten und gegebenenfalls am Carboxylende derivatisierten Aminosäure erleichtert,
   B) Kuppeln einer N-geschützten und gegebenenfalls am Carboxylende derivatisierten Aminosäure an den funktionalisierten Polystyrolbestandteil, wobei die Funktionalität und die N-geschützte und gegebenenfalls am Carboxylende derivatisierte Aminosäure so aufeinander eingestellt sind, daß die gebildete verankernde Bindung anschließend im wesentlichen ohne Abbau der zu synthetisierenden Peptid- oder Proteinkette gespalten werden kann,
   C) Entfernen der N-Schutzgruppe von einer N-geschützten Amino- oder substituierten Aminogruppe der gekuppelten und N-geschützten Aminosäure, so daß die Reaktion der Amino- oder substituierten Aminogruppe der gekuppelten Aminosäure mit einer Carboxylgruppe oder einer aktivierten Carboxylgruppe einer weiteren Aminosäure erleichtert ist,
   D) Umsetzen der Amino- oder substituierten Aminogruppe der zuletzt gekuppelten Aminosäure mit einer Carboxylgruppe oder einer aktivierten Carboxylgruppe einer weiteren N-geschützten Aminosäure, um eine Peptidbindung zwischen den beiden Aminosäurebestandteilen zu bilden,
   E) gegebenenfalls Entfernen der N-Schutzgruppe von einer N-geschützten Amino- oder substituierten Aminogruppe der zuletzt gekuppelten N-geschützten Aminosäure, so daß die Reaktion der Amino- oder substituierten Aminogruppe der letzteren Aminosäure mit einer Carboxylgruppe oder aktivierten Carboxylgruppe einer weiteren N-geschützten Aminosäure erleichtert ist,

F) in den Fällen, in denen Schritt E) ausgeführt worden ist, Wiederholen der Schritte D) und E) sooft dies gewünscht ist,

G) gegebenenfalls Entfernen von einigen oder allen Schutzgruppen, die möglicherweise an den Aminosäurebestandteilen der synthetisierten Peptid- oder Proteinkette verblieben sind,

H) gegebenenfalls Spalten der Bindung, welche die synthetisierte Peptid- oder Proteinkette mit dem funktionalisierten Polystyrolbestandteil verankert,

und

I) gegebenenfalls Entfernen jeder weiteren unerwünschten Gruppe von der synthetisierten Peptid- oder Proteinkette.

2. Verfahren zur Synthese von einem oder mehreren Peptiden oder Proteinen, welches, wenn zwei oder mehrere Peptide oder Proteine synthetisiert werden sollen, die parallele und im wesentlichen gleichzeitige Synthese der gewünschten Anzahl von Peptiden und Proteinen ermöglicht, wobei das Verfahren umfaßt:

A) Bereitstellen einer Vielzahl von im wesentlichen identischen Polymersubstraten, die mit Polystyrolketten gepfropft sind, wobei die Polystyrolketten gegebenenfalls weiter Substituenten tragen, die unter den in der Synthese vorherrschenden Bedingungen nicht reagieren, wobei das geschätzte Peak-Molekulargewicht der auf das Polymer gepfropften Polystyrolketten ohne Einschluß der gegebenenfalls vorhandenen Substituenten, mindestens 200.000 beträgt, wobei mindestens ein Teil der Polystyrolketten von jedem mit Polystyrol gepfropften Polymersubstrat mit einer chemischen Funktionalität funktionalisiert sind, welche die Bildung einer verankernden Brücke zwischen den Polystyrolbestandteilen und einer mindestens N-geschützten und gegebenenfalls am Carboxylende derivatisierten Aminosäure erleichtert,

B) gegebenenfalls das physikalische Auftrennen der Mitglieder der Vielzahl von mit Polystyrol gepfropften Polymersubstraten in zwei oder mehrere Gruppen, von denen jede eines oder mehrere Mitglieder der Vielzahl umfaßt, Kuppeln einer N-geschützten und gegebenenfalls am Carboxylende derivatisierten Aminosäure an die funktionalisierten Polystyrolbestandteile jedes Mitglieds der Vielzahl oder, soweit anwendbar, jedes Mitglieds jeder Gruppe, wobei die eingesetzte N-geschützte und gegebenenfalls am Carboxylende derivatisierte Aminosäure für alle Mitglieder der Vielzahl oder, soweit anwendbar, alle Mitglieder einer Gruppe identisch ist, und, soweit anwendbar, darüber hinaus mit einer der folgenden Alternativen übereinstimmt:

i) identisch für alle Gruppen,

ii) wenn die Anzahl der Gruppen größer als zwei ist, identisch für mindestens zwei der Gruppen,

iii) verschieden für jede Gruppe,

wobei die Funktionalität und die N-geschützte und gegebenenfalls am Carboxylende derivatisierte Aminosäure so aufeinander eingestellt sind, daß die gebildete verankernde Bindung anschließend im wesentlichen ohne Abbau der zu synthetisierenden Peptid- oder Proteinkette gespalten werden kann,

C) Behandeln jedes Mitglieds der Vielzahl oder, soweit anwendbar, jedes Mitglieds jeder Gruppe, um die N-Schutzgruppe von einer N-geschützten Amino- oder substituierten Aminogruppe der gekuppelten und N-geschützten Aminosäure zu entfernen, so daß die Reaktion der Amino- oder substituierten Aminogruppe der gekuppelten Aminosäure mit einer Carboxylgruppe oder einer aktivierten Carboxylgruppe einer weiteren N-geschützten Aminosäure erleichtert ist,

D) Umsetzen der Amino- oder substituierten Aminogruppe der Aminosäure, die zuletzt an die funktionalisierten Polystyrolbestandteile jedes Mitglieds der Vielzahl oder, soweit anwendbar, jedes Mitglieds jeder Gruppe gekuppelt worden ist, mit einer Carboxylgruppe oder einer aktivierten Carboxylgruppe einer weiteren N-geschützten Aminosäure, so daß eine Peptidbindung zwischen der Amino- oder substituierten Aminogruppe und der Carboxylgruppe oder aktivierten Carboxylgruppe gebildet wird, wobei die weitere N-geschützte Aminosäure für alle Mitglieder der Vielzahl oder, soweit anwendbar, alle Mitglieder einer Guppe identisch ist und, soweit anwendbar, ferner mit einer der drei oben im Zusammenhang mit Schritt B) erwähnten Alternativen übereinstimmt,

E) gegebenenfalls Behandeln jedes Mitglieds der Vielzahl oder, soweit anwendbar, jedes Mitglieds jeder Gruppe, um die N-Schutzgruppe von einer N-geschützten Amino- oder substituierten Aminogruppe der zuletzt gekuppelten N-geschützten Aminosäure zu entfernen, so daß die Reaktion der Amino- oder substituierten Aminogruppe der letzteren Aminosäure mit einer Carboxylgruppe oder aktivierten Carboxylgruppe einer weiteren N-geschützten Aminosäure erleichtert ist,

F) in den Fällen, in denen Schritt E) ausgeführt worden ist, Wiederholen der Schritte D) und E), sooft dies gewünscht wird,

G) gegebenenfalls Behandeln jedes Mitglieds der Vielzahl oder, soweit anwendbar, jedes Mitglieds jeder Gruppe, um einige oder alle Schutzgruppen zu entfernen, die möglicherweise an den Aminosäurebestandteilen der synthetisierten Peptid- oder Proteinkette verblieben sind,

H) gegebenenfalls Behandeln jedes Mitglieds der Vielzahl oder, soweit anwendbar, jedes Mitglieds jeder Gruppe, um die Bindung zu spalten, welche die synthetisierte Peptid- oder Proteinkette mit den funktionalisierten Polystyrolbestandteilen jedes Mitglieds der Vielzahl oder, soweit anwendbar, jedes Mitglieds jeder Gruppe verankert, und

I) gegebenenfalls Entfernen jeder weiteren unerwünschten Gruppe von einer synthetisierten Peptid- oder Proteinkette.

3. Verfahren zur Herstellung eines mit Polystyrol gepfropften Polymersubstrats, welches ein Peptid oder Protein trägt, welches darauf synthetisiert worden ist, wobei das Verfahren die Schritte umfaßt:

A) Bereitstellen eines mit Polystyrolketten gepfropften Polymersubstrats, wobei die Polystyrolketten gegebenenfalls weiter Substituenten tragen, welche unter den in der Synthese vorherrschenden Bedingungen nicht reagieren, wobei das geschätzte Peak-Molekulargewicht der auf das Polymer gepfropften Polystyrolketten ohne Einschluß gegebenenfalls vorhandener Substituenten, mindestens 200.000 beträgt, wobei mindestens ein Teil der Polystyrolketten des mit Polystyrol gepfropften Polymersubstrats mit einer chemischen Funktionalität funktionalisiert sind, welche die Bildung einer verankernden Bindung zwischen dem Polystyrolbestandteil und einer mindestens N-geschützten und gegebenenfalls am Carboxylende derivatisierten Aminosäure erleichtert,

B) Kuppeln einer N-geschützten und gegebenenfalls am Carboxylende derivatisierten Aminosäure an den funktionalisierten Polystyrolbestandteil, wobei die Funktionalität und die N-geschützte und gegebenenfalls am Carboxylende derivatisierte Aminosäure so aufeinander eingestellt sind, daß die gebildete verankernde Bindung anschließend im wesentlichen ohne Abbau der zu synthetisierenden Peptid- oder Proteinkette gespalten werden kann,

C) Entfernen der N-Schutzgruppe von einer N-geschützten Amino- oder substituierten Aminogruppe der gekuppelten und N-geschützten Aminosäure, so daß die Reaktion der Amino- oder substituierten Aminogruppe der gekuppelten Aminosäure mit einer Carboxylgruppe oder einer aktivierten Carboxylgruppe einer weiteren Aminosäure erleichtert ist,

D) Umsetzen der Amino- oder substituierten Aminogruppe der zuletzt gekuppelten Aminosäure mit einer Carboxylgruppe oder einer aktivierten Carboxylgruppe einer weiteren N-geschützten Aminosäure, um eine Peptidbindung zwischen den zwei Aminosäurebestandteilen zu bilden,

E) gegebenenfalls Entfernen der N-Schutzgruppe von einer N-geschützten Amino- oder substituierten Aminogruppe der zuletzt gekuppelten N-geschützten Aminosäure, so daß die Reaktion der Amino- oder substituierten Aminogruppe der letzteren Aminosäure mit einer Carboxylgruppe oder aktivierten Carboxylgruppe einer weiteren N-geschützten Aminosäure erleichtert ist,

F) in den Fällen, in denen Schritt E) ausgeführt worden ist, Wiederholen der Schritte D) und E), sooft dies gewünscht ist, und

G) gegebenenfalls Entfernen von einigen oder allen Schutzgruppen, die möglicherweise an den Aminosäurebestandteilen der synthetisierten Peptid- oder Proteinkette verblieben sind.

4. Verfahren nach einem der Ansprüche 1 bis 3, worin eines der mit Polystyrol gepfropften Polymersubstrate durch eine im wesentlichen radikal-gestartete Reaktion zwischen dem Polymersubstrat und gegebenenfalls substituiertem Styrolmonomer, welches in einer Lösung des Monomers in einem organischen Lösungsmittel vorhanden ist, gebildet wurde.

5. Verfahren nach Anspruch 4, worin das organische Lösungsmittel, welches verwendet wurde, um das gegebenenfalls substituierte Styrolmonomer aufzulösen, ein Alkohol ist.

6. Verfahren nach Anspruch 5, worin der Alkohol Methanol ist.

7. Verfahren nach Anspruch 6, worin der Volumenprozentsatz (% Vol./Vol.) von gegebenenfalls substituiertem Styrol in der Lösung, welche verwendet wurde, so ist, daß $25 \leq \%$ Vol./Vol. $\leq 35$.

8. Verfahren nach einem der vorhergehenden Ansprüche, worin das geschätzte Peak-Molekulargewicht der auf das Polymer gepfropften Polystyrolketten, ohne Einschluß von gegebenenfalls vorhandenen

EP 0 433 345 B1

Substituenten, im Bereich von 300.000 bis 1.600.000 liegt.

9. Verfahren nach einem der vorhergehenden Ansprüche, worin das mit Polystyrol gepfropfte Polymersubstrat aus einem Polymersubstrat in Form einer Folie oder eines Films mit einer Dicke im Bereich von 25 bis 100 μm gebildet worden ist, und das Ausmaß der Polystyrolkettenpfropfung des Polymersubstrats im Bereich von 5 bis 800 Gew.-% liegt.

10. Verfahren nach Anspruch 9, worin das Ausmaß der Polystyrolkettenpfropfung des Polymersubstrats im Bereich von 100 bis 600 Gew.-% liegt.

11. Verfahren nach einem der vorhergehenden Ansprüche, worin das mit Polystyrol gepfropfte Polymersubstrat in Form einer Folie oder eines Films vorliegt.

12. Verfahren nach einem der Ansprüche 1 bis 8, worin das mit Polystyrol gepfropfte Polymersubstrat aus einem Polymersubstrat in Form einer Kugel, eines Pellets, einer Scheibe, eines Rings, eines Rohrs, eines Stabs oder eines Netzes hergestellt wurde.

13. Verfahren nach einem der vorhergehenden Ansprüche, worin die chemische Funktionalität, welche die Bildung einer verankernden Bindung zwischen einer mindestens N-geschützten und gegebenenfalls derivatisierten Aminosäure und dem funktionalisierten Polystyrolbestandteil erleichtert
ein chlor-, brom- oder iod-substituiertes Alkyl,
ein amino-substituiertes Alkyl,
ein amino- und aryl-substituiertes Alkyl,
ein amino- und alkylaryl-substituiertes Alkyl, oder
ein hydroxy-substituiertes Alkyl
ist oder davon abgeleitet ist, wobei die Funktionalität, falls sie von einem dieser abgeleitet ist, eine Funktionalität mit einer Abstandshaltergruppe ist, so daß eine synthetisierte Peptid- oder Proteinkette von dem Polystyrolbestandteil im wesentlichen ohne Abbau der Kette abgespalten werden kann.

14. Verfahren nach Anspruch 13, worin die chemische Funktionalität, welche die Bildung einer verankernden Bindung erleichtert,
Chlormethyl,
Aminomethyl,
α-Aminobenzyl,
α-Amino-2-, α-Amino-3- oder α-Amino-4-methylbenzyl, oder
Hydroxymethyl
ist oder davon abgeleitet ist.

15. Verfahren nach einem der vorhergehenden Ansprüche, worin das Polymer ein Polyolefin ist.

16. Verfahren nach Anspruch 15, worin das Polyolefin Polyethylen ist.

17. Peptid- oder Protein-tragendes mit Polystyrol gepfropftes Polymersubstrat, welches durch ein Verfahren nach einem der Ansprüche 3 bis 16 hergestellt ist.

18. Polymersubstrat, welches mit Polystyrolketten gepfropft ist, und an welches ein Peptid oder Protein gekuppelt ist, wobei die Polystyrolketten gegebenenfalls weiter Substituenten tragen, welche unter den in der Peptidsynthese vorherrschenden Bedingungen nicht reagieren, wobei das geschätzte Peak-Molekulargewicht der auf das Polymer gepfropften Polystyrolketten ohne Einschluß gegebenenfalls vorhandener Substituenten mindestens 200.000 ist, wobei mindestens ein Teil der Polystyrolketten des mit Polystyrol gepfropften Polymersubstrats eine verankernde Bindung tragen, über die das Peptid oder Protein gekuppelt ist.

19. Mit Polystyrol gepfropftes Polymersubstrat nach Anspruch 18, wobei das geschätzte Peak-Molekulargewicht der auf das Polymer gepfropften Polystyrolketten ohne Einschluß gegebenenfalls vorhandener Substituenten im Bereich von 300.000 bis 1.600.000 liegt.

31

**20.** Mit Polystyrol gepfropftes Polymersubstrat nach Anspruch 18 oder 19, welches aus einem Polymersubstrat in Form einer Folie oder eines Films mit einer Dicke im Bereich von 25 bis 100 $\mu$m hergestellt wurde, und in welchem das Ausmaß der Polystyrolkettenpfropfung des Polymersubstrats im Bereich von 5 bis 800 Gew.-% liegt.

**21.** Mit Polystyrol gepfropftes Polymersubstrat nach Anspruch 20, worin das Ausmaß der Polystyrolkettenpfropfung des Polymersubstrats im Bereich von 100 bis 600 Gew.-% liegt.

**22.** Mit Polystyrol gepfropftes Polymersubstrat nach einem der Ansprüche 18 bis 21, welches in Form einer Folie oder eines Films vorliegt.

**23.** Mit Polystyrol gepfropftes Polymersubstrat nach Anspruch 18 oder 19, welches aus einem Polymersubstrat in Form einer Kugel, eines Pellets, einer Scheibe, eines Rings, eines Rohrs, eines Stabs oder eines Netzes hergestellt wurde.

**24.** Mit Polystyrol gepfropftes Polymersubstrat nach einem der Ansprüche 18 bis 23, worin das Polymer ein Polyolefin ist.

**25.** Mit Polystyrol gepfropftes Polymersubstrat nach Anspruch 24, worin das Polyolefin Polyethylen ist.

**26.** Polymersubstrat, das mit Polystyrolketten gepfropft ist, wobei die Polystyrolketten gegebenenfalls weiter Substituenten tragen, die unter den in der Peptidsynthese vorherrschenden Bedingungen nicht reagieren, wobei das geschätzte Peak-Molekulargewicht der auf das Polymer gepfropften Polystyrolketten ohne Einschluß gegebenenfalls vorhandener Substituenten mindestens 200.000 ist, wobei mindestens ein Teil der Polystyrolketten des mit Polystyrol gepfropften Polymersubstrats mit einer chemischen Funktionalität funktionalisiert sind, welche die Bildung einer verankernden Bindung zwischen dem Polystyrolbestandteil und einer mindestens N-geschützten und gegebenenfalls am Carboxylende derivatisierten Aminosäure erleichtert.

**27.** Mit Polystyrol gepfropftes Polymersubstrat nach Anspruch 26, worin das geschätzte Peak-Molekulargewicht der auf das Polymer gepfropften Polystyrolketten ohne Einschluß gegebenenfalls vorhandener Substituenten im Bereich von 300.000 bis 1.600.000 liegt.

**28.** Mit Polystyrol gepfropftes Polymersubstrat nach Anspruch 26 oder 27, welches aus einem Polymersubstrat in Form einer Folie oder eines Films mit einer Dicke im Bereich von 25 bis 100 $\mu$m hergestellt wurde, und in welchem das Ausmaß der Polystyrolkettenpfropfung des Polymersubstrats im Bereich von 5 bis 800 Gew.-% liegt.

**29.** Mit Polystyrol gepfropftes Polymersubstrat nach Anspruch 28, worin das Ausmaß der Polystyrolkettenpfropfung des Polymersubstrats im Bereich von 100 bis 600 Gew.-% liegt.

**30.** Mit Polystyrol gepfropftes Polymersubstrat nach einem der Ansprüche 26 bis 29, welches in Form einer Folie oder eines Films vorliegt.

**31.** Mit Polystyrol gepfropftes Polymersubstrat nach Anspruch 26 oder 27, welches aus einem Polymersubstrat in Form einer Kugel, eines Pellets, einer Scheibe, eines Rings, eines Rohrs, eines Stabs oder eines Netzes hergestellt wurde.

**32.** Mit Polystyrol gepfropftes Polymersubstrat nach einem der Ansprüche 26 bis 31, worin die chemische Funktionalität, welche die Bildung einer verankernden Bindung zwischen einer mindestens N-geschützten und gegebenenfalls derivatisierten Aminosäure und dem funktionalisierten Polystyrolbestandteil erleichtert,
ein chlor-, brom- oder iod-substituiertes Alkyl,
ein amino-substituiertes Alkyl,
ein amino- und aryl-substituiertes Alkyl,
ein amino- und alkylaryl-substituiertes Alkyl, oder
ein hydroxy-substituiertes Alkyl
ist oder davon abgeleitet ist, wobei die Funktionalität, wenn sie von einem dieser abgeleitet ist, eine

EP 0 433 345 B1

Funktionalität mit einer Abstandshaltergruppe ist, so daß eine synthetisierte Peptid- oder Proteinkette von dem Polystyrolbestandteil im wesentlichen ohne Abbau der Kette abgespalten werden kann.

33. Mit Polystyrol gepfropftes Polymersubstrat nach Anspruch 32, worin die chemische Funktionalität, welche die Bildung einer verankernden Brücke erleichtert,
Chlormethyl,
Aminomethyl,
α-Aminobenzyl,
α-Amino-2-, α-Amino-3-, oder α-Amino-4-methylbenzyl, oder
Hydroxymethyl
ist oder davon abgeleitet ist.

34. Mit Polystyrol gepfropftes Polymersubstrat nach Anspruch 32 oder 33, worin die Funktionalität von einem aminogruppenhaltigen Bestandteil abgeleitet ist, der ausgewählt ist aus: amino-substituiertem Alkyl; amino- und aryl-substituiertem Alkyl; und amino- und alkylarylsubstituiertem Alkyl; und die Funktionalität eine Abstandshaltergruppe umfaßt, die abgeleitet ist von einer niederen 4-(Halogenalkyl)-aryl-alkansäure, einer niederen Boc-aminoacyl-4-(oxymethyl)-aryl-alkansäure, einem N-Boc-p-acylbenzhydrylamin, einem N-Boc-4'-nieder-alkyl-p-acylbenzhydrylamin, einem N-Boc-4'-nieder-alkoxy-p-acylbenzhydrylamin oder einer niederen 4-Hydroxymethylphenoxy-alkansäure.

35. Mit Polystyrol gepfropftes Polymersubstrat nach einem der Ansprüche 26 bis 34, worin das Polymer ein Polyolefin ist.

36. Mit Polystyrol gepfropftes Polymersubstrat nach Anspruch 35, worin das Polyolefin Polyethylen ist.

37. Verfahren zur Herstellung eines funktionalisierten, mit Polystyrol gepfropften Polymersubstrats nach einem der Ansprüche 26 bis 36, umfassend das Unterziehen eines in eine Lösung aus gegebenenfalls substituiertem Styrolmonomer in einem organischen Lösungsmittel eingetauchten Polymersubstrats einer Behandlung, die zur Bildung von freien Radikalen führt, so daß Polystyrolketten auf das Polymersubstrat aufgepfropft werden, und das Funktionalisieren von mindestens einem Teil der Polystyrolketten mit einer chemischen Funktionalität, welche die Bildung einer verankernden Bindung zwischen dem Polystyrolbestandteil und einer mindestens N-geschützten und gegebenenfalls am Carboxylende derivatisierten Aminosäure erleichtert.

38. Verfahren zur Herstellung eines mit Polystyrolketten gepfropften Polymersubstrates, an welches eine mindestens N-geschützte Aminosäure gekuppelt ist, wobei die Polystyrolketten gegebenenfalls weiter Substituenten tragen, die unter den in der Peptidsynthese vorherrschenden Bedingungen nicht reagieren, wobei das geschätzte Peak-Molekulargewicht der auf das Polymer gepfropften Polystyrolketten ohne Einschluß gegebenenfalls vorhandener Substituenten mindestens 200.000 ist, wobei mindestens ein Teil der Polystyrolketten des mit Polystyrol gepfropften Polymersubstrats eine verankernde Bindung tragen, über welche die mindestens N-geschützte Aminosäure gekuppelt ist, wobei das Verfahren umfaßt das Unterziehen eines in eine Lösung von gegebenenfalls substituiertem Styrolmonomer in einem organischen Lösungsmittel eingetauchten Polymersubstrats einer Behandlung, welche zur Bildung von freien Radikalen führt, so daß Polystyrolketten auf das Polymersubstrat aufgepfropft werden, das Funktionalisieren von mindestens einem Teil der Polystyrolketten mit einer chemischen Funktionalität, welche die Bildung einer verankernden Bindung zwischen dem Polystyrolbestandteil und einer mindestens N-geschützten und gegebenenfalls am Carboxylende derivatisierten Aminosäure erleichtert, und das Reagierenlassen der Funktionalität, so daß eine verankernde Bindung zu einer mindestens N-geschützten Aminosäure gebildet wird.

39. Verfahren nach Anspruch 37 oder 38, worin die Behandlung, die zu der Bildung von freien Radikalen führt, Gammabestrahlung ist.

40. Verfahren nach Anspruch 39, worin die Gammabestrahlung durchgeführt wird unter Verwendung einer Gammastrahlungsdosisleistung von etwa 300 bis etwa 1000 Gy/Stunde.

41. Verfahren nach einem der Ansprüche 37 bis 40, worin das organische Lösungsmittel ein Alkohol ist.

33

**42.** Verfahren nach Anspruch 41, worin der Alkohol Methanol ist.

**43.** Verfahren nach einem der Ansprüche 37 bis 42, worin der Volumenprozentsatz (% Vol./Vol.) von gegebenenfalls substituiertem Styrol in der Lösung so ist, daß 25 ≤ % Vol./Vol. ≤ 35.

**44.** Verfahren nach einem der Ansprüche 37 bis 43, worin das Pfropfen so durchgeführt wird, daß sich ein geschätztes Peak-Molekulargewicht der auf das Polymer aufgepropften Polystyrolketten ohne Einschluß gegebenenfalls vorhandener Substituenten im Bereich von 300.000 bis 1.600.000 ergibt.

**45.** Verfahren nach einem der Ansprüche 37 bis 44, worin das Polymersubstrat in Form einer Folie oder eines Films mit einer Dicke im Bereich von 25 bis 100 $\mu$m vorliegt, und das Pfropfen so durchgeführt wird, daß sich ein Ausmaß der Polystyrolkettenpfropfung des Polymersubstrats im Bereich von 5 bis 800 Gew.-% ergibt.

**46.** Verfahren nach Anspruch 45, worin das Pfropfen so durchgeführt wird, daß sich ein Ausmaß der Polystyrolkettenpfropfung des Polymersubstrats im Bereich von 100 bis 600 Gew.-% ergibt.

**47.** Verfahren nach einem der Ansprüche 37 bis 44, worin das Polymersubstrat in Form einer Folie oder eines Films vorliegt.

**48.** Verfahren nach Anspruch 47, worin das Polymersubstrat eine Dicke von 25 bis 100 $\mu$m hat.

**49.** Verfahren nach einem der Ansprüche 37 bis 44, worin das Polymersubstrat in Form einer Kugel, eines Pellets, einer Scheibe, eines Rings, eines Rohrs, eines Stabs oder eines Netzes vorliegt.

**50.** Verfahren nach einem der Ansprüche 37 bis 49, worin die chemische Funktionalität, welche die Bildung einer verankernden Bindung zwischen einer mindestens N-geschützten und gegebenenfalls derivatisierten Aminosäure und dem funktionalisierten Polystyrolbestandteil erleichtert
ein chlor-, brom- oder iod-substituiertes Alkyl,
ein amino-substituiertes Alkyl,
ein amino- und aryl-substituiertes Alkyl,
ein amino- und alkylaryl-substituiertes Alkyl, oder
ein hydroxy-substituiertes Alkyl
ist oder davon abgeleitet ist, wobei die Funktionalität, wenn sie von einem dieser abgeleitet ist, eine Funktionalität mit einer Abstandshaltergruppe ist, so daß eine synthetisierte Peptid- oder Proteinkette von dem Polystyrolbestandteil im wesentlichen ohne Abbau der Kette abgespalten werden kann.

**51.** Verfahren nach Anspruch 50, worin die chemische Funktionalität, welche die Bildung einer verankernden Bindung erleichtert,
Chlormethyl,
Aminomethyl,
$\alpha$-Aminobenzyl,
$\alpha$-Amino-2-, $\alpha$-Amino-3- oder $\alpha$--Amino-4-methylbenzyl, oder
Hydroxymethyl
ist oder davon abgeleitet ist.

**52.** Verfahren nach Anspruch 37 oder 38, worin die Funktionalität abgeleitet ist von einem aminogruppenhaltigen Bestandteil, ausgewählt aus: amino-substituiertem Alkyl; amino- und aryl-substituiertem Alkyl; und amino- und alkylaryl-substituiertem Alkyl; und die Funktionalität behandelt wird, um die Funktionalität mit einer Abstandshaltergruppe zu versehen, die abgeleitet ist von einer niederen 4-(Halogenalkyl)-aryl-alkansäure, einer niederen Boc-aminoacyl-4-(oxymethyl)aryl-alkansäure, einem N-Boc-p-acylbenzhydrylamin, einem H-Boc-4'-niederalkyl-p-acylbenzhydrylamin, einem N-Boc-4'-niederalkoxy-p-acylbenzhydrylamin oder einer niederen 4-Hydroxymethylphenoxy-alkansäure.

**53.** Verfahren nach einem der Ansprüche 37 bis 52, worin das Polymer ein Polyolefin ist.

**54.** Verfahren nach Anspruch 53, worin das Polyolefin Polyethylen ist.

**55.** Polymersubstrat, gepropft mit Polystyrolketten und an das eine mindestens N-geschützte Aminosäure gekuppelt ist, wobei die Polystyrolketten gegebenenfalls weiter Substituenten tragen, die unter den in der Peptid-Synthese vorherrschenden Bedingungen nicht reagieren, wobei das geschätzte Peak-Molekulargewicht der auf das Polymer gepropften Polystyrolketten ohne Einschluß gegebenenfalls vorhandener Substituenten mindestens 200.000 beträgt, wobei mindestens ein Teil der Polystyrolketten des mit Polystyrol gepropften Polymersubstrats eine verankernde Bindung tragen, über welche die mindestens N-geschützte Aminosäure gekuppelt ist.

**56.** Mit Polystyrol gepropftes Polymersubstrat nach Anspruch 55, wobei das geschätzte Peak-Molekulargewicht der auf das Polymer gepropften Polystyrolketten ohne Einschluß gegebenenfalls vorhandener Substituenten im Bereich von 300.000 bis 1.600.000 liegt.

**57.** Mit Polystyrol gepropftes Polymersubstrat nach Anspruch 55 oder 56, welches aus einem Polymersubstrat in Form einer Folie oder eines Films mit einer Dicke im Bereich von 25 bis 100 μm hergestellt wurde, und in welchem das Ausmaß der Polystyrolkettenpfropfung des Polymersubstrats im Bereich von 5 bis 800 Gew.-% liegt.

**58.** Mit Polystyrol gepropftes Polymersubstrat nach Anspruch 57, worin das Ausmaß der Polystyrolkettenpfropfung des Polymersubstrats im Bereich von 100 bis 600 Gew.-% liegt.

**59.** Mit Polystyrol gepropftes Polymersubstrat nach einem der Ansprüche 55 bis 58, welches in Form einer Folie oder eines Films vorliegt.

**60.** Mit Polystyrol gepropftes Polymersubstrat nach Anspruch 55 oder 56, welches aus einem Polymersubstrat in Form einer Kugel, eines Pellets, einer Scheibe, eines Rings, eines Rohrs, eines Stabs oder eines Netzes hergestellt wurde.

**61.** Mit Polystyrol gepropftes Polymersubstrat nach einem der Ansprüche 55 bis 60, worin das Polymer ein Polyolefin ist.

**62.** Mit Polystyrol gepropftes Polymersubstrat nach Anspruch 61, worin das Polyolefin Polyethylen ist.

**63.** Polymersubstrat, welches mit Polystyrolketten gepropft ist, wobei die Polystyrolketten gegebenenfalls weiter Substituenten tragen, wobei das geschätzte Peak-Molekulargewicht der auf das Polymer gepropften Polystyrolketten ohne Einschluß gegebenenfalls vorhandener Substituenten mindestens 200.000 ist.

**64.** Mit Polystyrol gepropftes Polymersubstrat nach Anspruch 63, worin das geschätzte Peak-Molekulargewicht der auf das Polymer gepropften Polystyrolketten ohne Einschluß gegebenenfalls vorhandener Substituenten im Bereich von 300.000 bis 1.600.000 liegt.

**65.** Mit Polystyrol gepropftes Polymersubstrat nach Anspruch 63 oder 64, welches aus einem Polymersubstrat in Form einer Folie oder eines Films mit einer Dicke im Bereich von 25 bis 100 μm hergestellt wurde, und in welchem das Ausmaß der Polystyrolkettenpfropfung des Polymersubstrats im Bereich von 5 bis 800 Gew.-% liegt.

**66.** Mit Polystyrol gepropftes Polymersubstrat nach Anspruch 65, worin das Ausmaß der Polystyrolkettenpfropfung des Polymersubstrats im Bereich von 100 bis 600 Gew.-% liegt.

**67.** Mit Polystyrol gepropftes Polymersubstrat nach einem der Ansprüche 63 bis 66, welches in Form einer Folie oder eines Films vorliegt.

**68.** Mit Polystyrol gepropftes Polymersubstrat nach Anspruch 63 oder 64, welches aus einem Polymersubstrat in Form einer Kugel, eines Pellets, einer Scheibe, eines Rings, eines Rohrs, eines Stabs oder eines Netzes hergestellt wurde.

**69.** Mit Polystyrol gepropftes Polymersubstrat nach einem der Ansprüche 63 bis 68, worin das Polymer ein Polyolefin ist.

**70.** Mit Polystyrol gepfropftes Polymersubstrat nach Anspruch 69, worin das Polyolefin Polyethylen ist.

**71.** Verwendung eines Peptid-tragenden, mit Polystyrol gepfropften Polymersubstrats nach einem der Ansprüche 17 bis 25 in einem analytischen Verfahren als lichtdurchlässiges Trägermaterial, welches die spektralphotometrische Verfolgung einer Antigen/Antikörperreaktion erleichtert, wobei das Antigen ein Peptid ist, welches auf dem mit Polystyrol gepfropften Polymersubstrat synthetisiert wurde und daran verankert bleibt.

**72.** Verwendung nach Anspruch 71, wobei die Verfolgung die Verwendung einer ELISA-Methode nach sich zieht.

**Revendications**

**1.** Procédé pour la synthèse d'un peptide ou d'une protéine, ledit procédé comprenant les étapes de:

A) mise en oeuvre d'un substrat polymère greffé avec des chaînes de polystyrène, lesdites chaînes de polystyrène portant en outre en option des substituants qui ne sont pas réactifs dans les conditions régnant dans la synthèse, la masse moléculaire maximale estimée des chaînes de polystyrène greffées au polymère, non compris des substituants optionnels, étant d'au moins 200.000, au moins une partie des chaînes de polystyrène du substrat polymère greffé avec du polystyrène étant fonctionnalisées avec une fonctionnalité chimique facilitant la formation d'une liaison d'ancrage entre la portion polystyrène et un acide aminé, au moins N-protégé et en option converti en dérivé à une extrémité carboxyle,

B) couplage d'un acide aminé, N-protégé et en option converti en dérivé à une extrémité carboxyle, à la portion polystyrène fonctionnalisée, ladite fonctionnalité et ledit acide aminé, N-protégé et en option converti en dérivé à une extrémité carboxyle , étant adaptés l'un à l'autre de telle sorte que la liaison d'ancrage formée puisse ensuite être clivée pratiquement sans dégradation de la chaîne peptidique ou protéinique qui est à synthétiser,

C) élimination du groupe N-protecteur d'un groupe amino ou amino substitué N-protégé de l'acide aminé couplé et N-protégé, de telle sorte que la réaction du groupe amino ou amino substitué de l'acide aminé couplé avec un groupe carboxyle ou un groupe carboxyle activé d'un autre acide aminé soit facilitée,

D) réaction dudit groupe amino ou amino substitué de l'acide aminé couplé en dernier avec un groupe carboxyle ou un groupe carboxyle activé d'un autre acide aminé N-protégé de façon à former une liaison peptidique entre les deux portions acide aminé,

E) en option élimination du groupe N-protecteur d'un groupe amino ou amino substitué N-protégé de l'acide aminé N-protégé couplé en dernier, de telle sorte que la réaction du groupe amino ou amino substitué de ce dernier acide aminé avec un groupe carboxyle ou un groupe carboxyle activé d'un autre acide aminé N-protégé soit facilitée,

F) dans les cas où l'étape E) a été réalisée, répétition des étapes D) et E) un nombre de fois souhaité,

G) en option élimination de tout ou partie des groupes protecteurs éventuellement restants sur les portions acide aminé de la chaîne peptidique ou protéinique synthétisée,

H) en option coupure de la liaison d'ancrage de la chaîne peptidique ou protéinique synthétisée avec la portion de polystyrène fonctionnalisée,

et

I) en option élimination de tout autre groupe non-souhaité de la chaîne peptidique ou protéinique synthétisée.

**2.** Procédé pour la synthèse d'un ou plusieurs peptides ou protéines, lequel procédé, lorsque deux ou plus de deux peptides ou protéines sont à synthétiser, permet la synthèse parallèle et pratiquement simultanée du nombre souhaité de peptides et de protéines, ledit procédé comprenant:

A) la mise en oeuvre d'une pluralité de substrats polymères pratiquement identiques, greffés avec des chaînes de polystyrène, lesdites chaînes de polystyrène portant en outre en option des substituants qui ne sont pas réactifs dans les conditions régnant dans la synthèse, la masse moléculaire maximale estimée des chaînes de polystyrène greffées au polymère, non compris des substituants optionnels, étant d'au moins 200.000, au moins une partie des chaînes de polystyrène de chaque substrat polymère greffé avec du polystyrène étant fonctionnalisées avec une fonctionnalité chimique facilitant la formation d'une liaison d'ancrage entra les portions polystyrène et un acide aminé, au moins N-protégé et en option converti en dérivé à une extrémité carboxyle,

B) en option la séparation physique des membres de ladite pluralité de substrats de polymère greffé avec du polystyrène en deux ou plus de deux groupes dont chacun, comprend un ou plusieurs membres de ladite pluralité, le couplage d'un acide aminé, N-protégé et en option converti en dérivé à une extrémité carboxyle, aux fractions polystyrène fonctionnalisées de chaque membre de ladite pluralité ou, le cas échéant, chaque membre de chaque groupe, l'acide aminé, au moins N-protégé et en option converti dérivé à une extrémité carboxyle, employé étant identique pour tous les membres de la pluralité ou, le cas échéant, pour tous les membres d'un groupe, et, le cas échéant, étant en outre conforme à l'une des alternatives suivantes :

(i) identique pour tous les groupes,

(ii) lorsque le nombre desdits groupes est supérieur à deux, identique pour au moins deux des groupes,

(iii) différent pour chaque groupe;

ladite fonctionnalité et ledit acide aminé, N-protégé et en option converti en dérivé à une extrémité carboxyle, étant adaptés l'un à l'autre de telle sorte que la liaison d'ancrage formée puisse ensuite être clivée pratiquement sans dégradation de la chaîne peptidique ou protéinique qui est à synthétiser,

C) le traitement de chaque membre de ladite pluralité ou, le cas échéant , de chaque membre de chacun desdits groupes de façon à éliminer le groupe N-protecteur d'un groupe amino ou amino substitué N-protégé de l'acide aminé couplé et N-protégé, de telle sorte que la réaction du groupe amino ou amino substitué de l'acide aminé couplé avec un groupe carboxyle ou un groupe carboxyle activé d'un autre acide aminé N-protégé soit facilitée,

D) la réaction dudit groupe amino ou amino substitué de l'acide aminé couplé en dernier aux portions polystyrène fonctionnalisées de chaque membre de ladite pluralité ou, le cas échéant , de chaque membre de chaque groupe avec un groupe carboxyle ou un groupe carboxyle activé d'un autre acide aminé N-protégé, de façon à former une liaison peptidique entre ledit groupe amino ou amino substitué et ledit groupe carboxyle ou groupe carboxyle activé, ledit autre acide aminé N-protégé étant identique pour tous les membres de la pluralité ou, le cas échéant, tous les membres d'un groupe, et, le cas échéant, étant en outre conforme à l'une des trois alternatives mentionnées plus haut en relation avec l'étape B),

E) en option le traitement de chaque membre de ladite pluralité ou, le cas échéant, de chaque membre de chacun desdits groupes, de façon à éliminer le groupe N-protecteur d'un groupe amino ou amino substitué N-protégé de l'acide aminé N-protégé couplé en dernier, de telle sorte que la réaction du groupe amino ou amino substitué de ce dernier acide aminé avec un groupe carboxyle ou un groupe carboxyle activé d'un autre acide aminé N-protégé soit facilitée,

F) dans les cas où l'étape E) a été réalisée, la répétition des étapes D) et E) un nombre de fois souhaité,

G) en option le traitement de chaque membre de ladite pluralité ou, le cas échéant , de chaque membre de chacun desdits groupes de façon à éliminer tout ou partie des groupes protecteurs éventuellement restants sur les portions acide aminé de la chaîne peptidique ou protéinique synthétisée,

H) en option le traitement de chaque membre de ladite pluralité ou, le cas échéant , de chaque membre de chacun desdits groupes de façon à cliver la liaison d'ancrage de la chaîne peptidique ou protéinique synthétisée aux portions de polystyrène fonctionnalisées de chaque membre de ladite pluralité ou, le cas échéant, de chaque membre de chacun desdits groupes,

et

I) en option l'élimination de tout autre groupe non-souhaité d'une chaîne peptidique ou protéinique synthétisée.

3. Procédé pour la préparation d'un substrat polymère greffé avec du polystyrène portant un peptide ou une protéine, qui a été synthétisé sur lui, ledit procédé comprenant les étapes de:

A) mise en oeuvre d'un substrat polymère greffé avec des chaînes de polystyrène, lesdites chaînes de polystyrène portant en outre en option des substituants qui ne sont pas réactifs dans les conditions régnant dans la synthèse, la masse moléculaire maximale estimée des chaînes de polystyrène greffées au polymère, non compris des substituants optionnels, étant d'au moins 200.000, au moins une partie des chaînes de polystyrène du substrat polymère greffé avec du polystyrène étant fonctionnalisées avec une fonctionnalité chimique facilitant la formation d'une liaison d'ancrage entre la portion polystyrène et un acide aminé, au moins N-protégé et en option

EP 0 433 345 B1

converti en dérivé à une extrémité carboxyle,

B) couplage d'un acide aminé, N-protégé et en option converti en dérivé à une extrémité carboxyle, à la portion polystyrène fonctionnalisée, ladite fonctionnalité et ledit acide aminé, N-protégé et en option converti en dérivé à une extrémité carboxyle , étant adaptés l'un à l'autre de telle sorte que la liaison d'ancrage formée puisse ensuite être clivée pratiquement sans dégradation de la chaîne peptidique ou protéinique qui est à synthétiser,

C) élimination du groupe N-protecteur d'un groupe amino ou amino substitué N-protégé de l'acide aminé couplé et N-protégé, de telle sorte que la réaction du groupe amino ou amino substitué de l'acide aminé couplé avec un groupe carboxyle ou un groupe carboxyle activé d'un autre acide aminé soit facilitée,

D) réaction dudit groupe amino ou amino substitué de l'acide aminé couplé en dernier avec un groupe carboxyle ou un groupe carboxyle activé d'un autre acide aminé N-protégé de façon à former une liaison peptidique entre les deux portions acide aminé,

E) en option élimination du groupe N-protecteur d'un groupe amino ou amino substitué N-protégé de l'acide aminé N-protégé couplé en dernier, de telle sorte que la réaction du groupe amino ou amino substitué de ce dernier acide aminé avec un groupe carboxyle ou un groupe carboxyle activé d'un autre acide aminé N-protégé soit facilitée,

F) dans les cas où l'étape E) a été réalisée, répétition des étapes D) et E) un nombre de fois souhaité,

et

G) an option élimination de tout ou partie des groupes protecteurs éventuellement restants sur les portions acide aminé de la chaîne peptidique ou protéinique synthétisée.

4. Procédé selon l'une quelconque des revendications 1 à 3, dans lequel un substrat polymère greffé avec du polystyrène a été formé par une réaction pratiquement initiée par des radicaux entre le substrat polymère et le monomère styrène éventuellement substitué présent dans une solution dudit monomère dans un solvant organique.

5. Procédé selon la revendication 4, dans lequel le solvant organique qui a été utilisé pour dissoudre le monomère styrène éventuellement substitué est un alcool.

6. Procédé selon la revendication 5, dans lequel ledit alcool est le méthanol.

7. Procédé selon la revendication 6, dans lequel le pourcentage en volume (% en v/v) du styrène éventuellement substitué dans la solution oui a été utilisé est tel que $25 \leqq$ % en v/v $\leqq 35$.

8. Procédé selon l'une quelconque des revendications précédentes, dans lequel la masse moléculaire maximale estimée des chaînes de polystyrène greffées au polymère, non compris des substituants éventuels, est de 300.000 à 1.600.000.

9. Procédé selon l'une quelconque des revendications précédentes, dans lequel le substrat de polymère greffé avec du polystyrène a été préparé à partir d'un substrat polymère sous la forme d'une feuille ou d'une pellicule d'épaisseur de 25 à 100 $\mu$m, et le degré de greffage de chaînes de polystyrène du substrat polymère est de 5 à 800% en poids.

10. Procédé selon la revendication 9, dans lequel le degré de greffage de chaînes de polystyrène du substrat polymère est de 100 à 600% en poids.

11. Procédé selon l'une quelconque des revendications précédentes, dans lequel le substrat polymère greffé avec du polystyrène est sous la forme d'une feuille ou d'uns pellicule.

12. Procédé selon l'une quelconque des revendications 1 à 8, dans lequel le substrat de polymère greffé avec du polystyrène a été préparé à partir d'un substrat polymère sous la forme d'une bille , d'une pastille, d'un disques d'un anneau, d'un tube, d'une barre ou d'un filet.

13. Procédé selon l'une quelconque des revendications précédentes, dans lequel la fonctionnalité chimique facilitant la formation d'une liaison d'ancrage entre un acide aminé au moins N-protégé et en option converti en dérivé et la portion polystyrène fonctionnalisé est, ou est dérivée de,

38

un groupe alkyle chloro-, bromo- ou iodo-substitué,
un groupe alkyle amino-substitué,
un groupe alkyle amino- et aryl-substitué,
un groupe alkyle amino- et alkylaryl-substitué, ou
un groupe alkyle hydroxy-substitué,
la fonctionnalité, si elle dérive de l'un quelconque de ceux-ci, étant une fonctionnalité avec un groupe espaceur tel qu'une chaîne peptidique ou protéinique synthétisée puisse être clivée de la portion polystyrène pratiquement sans dégradation de ladite chaîne.

14. Procédé selon la revendication 13, dans lequel ladite fonctionnalité chimique facilitant la formation d'une liaison d'ancrage est, ou est dérivée de,
un groupe chlorométhyle,
un groupe aminométhyle,
un groupe $\alpha$-aminobenzyle,
un groupe $\alpha$-amino-2-, $\alpha$-amino-3- ou $\alpha$-amino-4-méthylbenzyle,
un groupe hydroxyméthyle.

15. Procédé selon l'une quelconque des revendications précédentes, dans lequel le polymère est une polyoléfine.

16. Procédé selon la revendication 15, dans lequel ladite polyoléfine est le polyéthylène.

17. Substrat polymère greffé avec du polystyrène portant un peptide ou une protéine, préparé par un procédé selon l'une quelconque des revendications 3 à 16.

18. Substrat polymère greffé avec des chaînes de polystyrène et auquel est couplé un peptide ou une protéine, lesdites chaînes de polystyrène portant en outre en option des substituants qui ne sont pas réactifs dans les conditions régnant dans la synthèse des peptides, la masse moléculaire maximale estimée des chaînes de polystyrène greffées au polymère, non compris des substituants éventuels, étant d'au moins 200.000, au moins une partie des chaînes de polystyrène du substrat polymère greffé avec du polystyrène portant une liaison d'ancrage via laquelle ledit peptide ou ladite protéine est couplé.

19. Substrat polymère greffé avec du polystyrène selon la revendication 18, dans lequel la masse moléculaire maximale estimée des chaînes de polystyrène greffées au polymère, non compris des substituants optionnels, est de 300.000 à 1.600.000.

20. Substrat polymère greffé avec du polystyrène selon l'une des revendications 18 ou 19, qui a été préparé à partir d'un substrat polymère sous la forme d'une feuille ou d'une pellicule ayant une épaisseur de 25 à 100 $\mu$m, et dans lequel le degré de greffage de chaînes de polystyrène du substrat polymère est de 5 à 800% en poids.

21. Substrat polymère greffé avec du polystyrène selon la revendication 20, dans lequel le degré de greffage de chaînes de polystyrène du substrat polymère est de 100 à 600 % en poids.

22. Substrat polymère greffé avec du polystyrène selon l'une quelconque des revendications 18 à 21, qui est sous la forme d'une feuille ou d'une pellicule.

23. Substrat polymère greffé avec du polystyrène selon l'une des revendications 18 ou 19, qui a été préparé à partir d'un substrat polymère sous la forme d'une bille, d'une pastille, d'un disque, d'un anneau, d'un tube, d'une barre ou d'un filet.

24. Substrat polymère greffé avec du polystyrène selon l'une quelconque des revendications 18 à 23, dans lequel le polymère est une polyoléfine.

25. Substrat polymère greffé avec du polystyrène selon la revendication 24, dans lequel ladite polyoléfine est le polyéthylène.

26. Substrat polymère greffé avec des chaînes de polystyrène, lesdites chaînes de polystyrène portant en outre en option des substituants qui ne sont pas réactifs dans les conditions régnant dans la synthèse des peptides, la masse moléculaire maximale estimée des chaînes de polystyrène greffées au polymère, non compris des substituants optionnels, étant d'au moins 200.000, au moins une partie des chaînes de polystyrène du substrat polymère greffé avec du polystyrène étant fonctionnalisées avec une fonctionnalité chimique facilitant la formation d'une liaison d'ancrage entre la portion polystyrène et un acide aminé, au moins N-protégé et en option converti en dérivé à une extrémité carboxyle.

27. Substrat polymère greffé avec du polystyrène selon la revendication 26, dans lequel la masse moléculaire maximale estimée des chaînes de polystyrène greffées sur le polymère, non compris des substituants optionnels, est de 300.000 à 1.600.000.

28. Substrat polymère greffé avec du polystyrène selon l'une des revendications 26 ou 27, qui a été préparé à partir d'un substrat polymère sous la forme d'une feuille ou d'une pellicule ayant une épaisseur de 25 à 100 μm, et dans lequel le degré de greffage de chaînes de polystyrène du substrat polymère est de 5 à 800% en poids.

29. Substrat polymère greffé avec du polystyrène selon la revendication 28, dans lequel le degré de greffage de chaînes de polystyrène du substrat polymère est de 100 à 600 % en poids.

30. Substrat polymère greffé avec du polystyrène selon l'une quelconque des revendications 26 à 29, qui est sous la forme d'une feuille ou d'une pellicule.

31. Substrat polymère greffé avec du polystyrène selon l'une des revendications 26 ou 27, qui a été préparé à partir d'un substrat polymère sous la forme d'une bille, d'une pastille, d'un disque, d'un anneau, d'un tube, d'une barre ou d'un filet.

32. Substrat polymère greffé avec du polystyrène selon l'une quelconque des revendications 26 à 31, dans lequel la fonctionnalité chimique facilitant la formation d'une liaison d'ancrage entre un acide aminé au moins N-protégé et en option converti en dérivé et la portion polystyr fonctionnalisée est, ou est dérivée de,
un groupe alkyle chloro-, bromo- ou iodo-substitué,
un groupe alkyle amino-substitué,
un groupe alkyle amino- et aryl-substitué,
un groupe alkyle amino- et alkylaryl-substitué, ou
un groupe alkyle hydroxy-substitué,
la fonctionnalité, si elle dérive de l'un quelconque de ceux-ci, étant une fonctionnalité avec un groupe espaceur tel qu'une chaîne peptidique ou protéinique synthétisée puisse être clivée de la portion polystyrène pratiquement sans dégradation de ladite chaîne.

33. Substrat polymère greffé avec du polystyrène selon la revendication 32, dans lequel ladite fonctionnalité chimique facilitant la formation d'une liaison d'ancrage est, ou est dérivée de,
un groupe chlorométhyle,
un groupe aminométhyle,
un groupe α-aminobenzyle,
un groupe α-amino-2-, α-amino-3- ou α-amino-4-méthylbenzyle,
ou un groupe hydroxyméthyle.

34. Substrat polymère greffé avec du polystyrène selon l'une des revendications 32 ou 33, dans lequel la fonctionnalité est dérivée d'un groupe contenant un groupe amino et choisi parmi les groupes: alkyle amino-substitué; alkyle amino- et aryl-substitué; et alkyle amino- et alkylaryl-substitué; et la fonctionnalité comprend un groupe espaceur dérivé d'un acide 4-(halogénoalkyl)aryl-alcane(inférieur)oïque, un acide Boc-aminoacyl-4-(oxyméthyl)aryl-alcane(inférieur)oïque, une N-Boc-p-acylbenzhydrylamine, une N-Boc-4'-alkyl inférieur-p-acylbenzhydrylamine, une N-Boc-4'-alcoxy inférieur-p-acylbenzhydrylamine ou un acide 4-hydroxyméthylphénoxy-alcane(inférieur)oïque.

35. Substrat polymère greffé avec du polystyrène selon l'une quelconque des revendications 26 à 34, dans lequel le polymère est une polyoléfine.

**36.** Substrat polymère greffé avec du polystyrène selon la revendication 35, dans lequel ladite polyoléfine est le polyéthylène.

**37.** Procédé pour la préparation d'un substrat polymère greffé avec du polystyrène fonctionnalisé selon l'une quelconque des revendications 26 à 36, comprenant le traitement d'un substrat polymère, immergé dans une solution de monomère de styrène éventuellement substitué dans un solvant organique, conduisant à la formation de radicaux libres tels que des chaînes de polystyrène soient greffées au substrat polymère, et la fonctionnalisation d'au moins une partie des chaînes de polystyrène avec une fonctionnalité chimique facilitant la formation d'une liaison d'ancrage entre la portion polystyrène et un acide aminé, au moins N-protégé et en option converti en dérive à une extrémité carboxyle.

**38.** Procédé pour la préparation d'un substrat polymère greffé avec des chaînes de polystyrène et auquel un acide aminé au moins N-protégé est couplé, lesdites chaînes de polystyrène portant en outre en option des substituants qui ne sont pas réactifs dans les conditions régnant dans la synthèse des peptides, la masse moléculaire maximale estimée des chaînes de polystyrène greffées au polymère, non compris des substituants optionnels, étant d'au moins 200.000, au moins une partie des chaînes de polystyrène du substrat polymère greffé avec du polystyrène portant une liaison d'ancrage via laquelle ledit acide aminé au moins N-protégé est couplé, ledit procédé comprenant le traitement d'un substrat polymère, immergé dans une solution de monomère de styrène éventuellement substitué dans un solvant organique, conduisant à la formation de radicaux libres tels que des chaînes de polystyrène soient greffées au substrat polymère, la fonctionnalisation d'au moins une partie des chaînes de polystyrène avec une fonctionnalité chimique facilitant la formation d'une liaison d'ancrage entre la portion polystyrène et un acide aminé, au moins N-protégé et en option converti en dérivé à une extrémité carboxyle, et la réaction de ladite fonctionnalité de façon à former une liaison d'ancrage avec un acide aminé au moins N-protégé.

**39.** Procédé selon l'une des revendications 37 ou 38, dans lequel le traitement conduisant à la formation de radicaux libres est une irradiation gamma.

**40.** Procédé selon la revendication 39, dans lequel l'irradiation gamma est réalisée avec une dose de rayonnement gamma d'environ 300 à 1.000 Gy/heure.

**41.** Procédé selon l'une quelconque des revendications 37 à 40, dans lequel le solvant organique est un alcool.

**42.** Procédé selon la revendication 41, dans lequel ledit alcool est le méthanol.

**43.** Procédé selon l'une quelconque des revendications 37 à 42, dans lequel le pourcentage en volume (% en v/v) du styrène éventuellement substitué dans la solution est tel que 25 ≤ % en v/v ≤ 35.

**44.** Procédé selon l'une quelconque des revendications 37 à 43, dans lequel le greffage est effectué de façon à procurer une masse moléculaire maximale estimée des chaînes de polystyrène greffées au polymère, non compris des substituants optionnels, de 300.000 à 1.600.000.

**45.** Procédé selon l'une queclconque des revendications 37 à 44, dans lequel le substrat polymère est sous la forme d'une feuille ou d'une pellicule ayant une épaiseur de 25 à 100 $\mu$m, et le greffage est effectué de facon à procurer un degré de greffage de chaînes de polystyrène du substrat polymère de 5 à 800% en poids.

**46.** Procédé selon la revendication 45, dans lequel le greffage est effectué de façon à procurer un degré de greffage de chaînes de polystyrène du substrat polymère de 100 à 600% en poids.

**47.** Procédé selon l'une quelconque des revendications 37 à 44, dans lequel le substrat polymère est sous la forme d'une feuille ou d'une pellicule.

**48.** Procédé selon la revendication 47, dans lequel le substrat polymère a une épaisseur de 25 à 100 $\mu$m.

**49.** Procédé selon l'une quelconque des revendications 37 à 44, dans lequel le substrat polymère est sous la forme d'une bille, d'une pastille, d'un disque, d'un anneau, d'un tube, d'une barre ou d'un filet.

**50.** Procédé selon l'une quelconque des revendications 37 à 49, dans lequel la fonctionnalité chimique facilitant la formation d'une liaison d'ancrage entre un acide aminé au moins N-protégé et en option converti en dérivé et la portion polystyrène fonctionnalisé est, ou est dérivée de,
un groupe alkyle chloro-, bromo- ou iodo-substitué,
un groupe alkyle amino-substitué,
un groupe alkyle amino- et aryl-substitué,
un groupe alkyle amino- et alkylaryl-substitué, ou
un groupe alkyle hydroxy-substitué,
la fonctionnalité, si elle dérive de l'un quelconque de ceux-ci, étant une fonctionnalité avec un groupe espaceur tel qu'une chaîne peptidique ou protéinique synthétisée puisse être clivée de la portion polystyrène pratiquement sans dégradation de ladite chaîne.

**51.** Procédé selon la revendication 50, dans lequel ladite fonctionnalité chimique facilitant la formation d'une liaison d'ancrage est, ou est dérivée de,
un groupe chlorométhyle,
un groupe aminométhyle,
un groupe α-aminobenzyle,
un groupe α-amino-2-, α-amino-3- ou α-amino-4-méthylbenzyle,
ou un groupe hydroxyméthyle.

**52.** Procédé selon l'une des revendications 37 ou 38, dans lequel la fonctionnalité est dérivée d'un groupe contenant un groupe amino et choisi parmi les groupes: alkyle amino-substitué; alkyle amino- et aryl-substitué; et alkyle amino- et alkylaryl-substitué; et la fonctionnalité est traitée pour procurer la fonctionnalité avec un groupe espaceur dérivé d'un acide 4-(halogénoalkyl)aryl-alcane(inférieur)oïque, un acide Boc-aminoacyl-4-(oxyméthyl)aryl-alcane(inférieur)oïque, une N-Boc-p-acylbenzhydrylamine, une N-Boc-4'-alkyl inférieur-p-acylbenzhydrylamine, une N-Boc-4'-alcoxy inférieur-p-acylbenzhydrylamine ou un acide 4-hydroxyméthylphénoxy-alcane(inférieur)oïque.

**53.** Procédé selon l'une quelconque des revendications 37 à 52, dans lequel le polymère est une polyoléfine.

**54.** Procédé selon la revendication 53, dans lequel ladite polyoléfine est le polyéthylène.

**55.** Substrat polymère greffé avec des chaînes de polystyrène et auquel un acide aminé au moins N-protégé est couplé, lesdites chaînes de polystyrène portant en outre en option des substituants qui ne sont pas réactifs dans les conditions régnant dans la synthèse des peptides, la masse moléculaire maximale estimée des chaînes de polystyrène greffées au polymère, non compris des substituants optionnels, étant d'au moins 200.000, au moins une partie des chaînes de polystyrène du substrat polymère greffé avec du polystyrène portant une liaison d'ancrage via laquelle ledit acide aminé au moins N-protégé est couplé.

**56.** Substrat polymère greffé avec du polystyrène selon la revendication 55, dans lequel la masse moléculaire maximale estimée des chaînes de polystyrène greffées au polymère, non compris des substituants optionnels, est de 300.000 à 1.600.000.

**57.** Substrat polymère greffé avec du polystyrène selon l'une des revendications 55 ou 56, qui a été préparé à partir d'un substrat polymère sous la forme d'une feuille ou d'une pellicule ayant une épaisseur de 25 à 100 μm, et dans lequel le degré de greffage de chaînes de polystyrène du substrat polymère est de 5 à 800% en poids.

**58.** Substrat polymère greffé avec du polystyrène selon la revendication 57, dans lequel le degré de greffage de chaînes de polystyrène du substrat polymère est de 100 à 600% en poids.

**59.** Substrat polymère greffé avec du polystyrène selon l'une quelconque des revendications 55 à 58, qui est sous la forme d'une feuille ou d'une pellicule.

**60.** Substrat polymère greffé avec du polystyrène selon l'une des revendications 55 ou 56, qui a été préparé à partir d'un substrat polymère sous la forme d'une bille, d'une pastille, d'un disque, d'un anneau, d'un tube, d'une barre ou d'un filet.

**61.** Substrat polymère greffé avec du polystyrène selon l'une quelconque des revendications 55 à 60, dans lequel le polymère est une polyoléfine.

**62.** Substrat polymère greffé avec du polystyrène selon la revendication 61, dans lequel la polyoléfine est le polyéthylène.

**63.** Substrat polymère greffé avec des chaînes de polystyrène, lesdites chaînes de polystyrène portant en outre en option des substituants, la masse moléculaire maximale estimée des chaînes de polystyrène greffées au polymère, non compris des substituants éventuels, étant d'au moins 200.000.

**64.** Substrat polymère greffé avec du polystyrène selon la revendication 63, dans lequel la masse moléculaire maximale estimée des chaînes de polystyrène greffées au polymère, non compris des substituants optionnels, est de 300.000 à 1.600.000.

**65.** Substrat polymère greffé avec du polystyrène selon l'une des revendications 63 ou 64, qui a été préparé à partir d'un substrat polymère sous la forme d'une feuille ou d'une pellicule ayant une épaisseur de 25 à 100 $\mu$m, et dans lequel le degré de greffage de chaînes de polystyrène du substrat polymère est de 5 à 800% en poids.

**66.** Substrat polymère greffé avec du polystyrène selon la revendication 65, dans lequel le degré de greffage de chaînes de polystyrène du substrat polymère est de 100 à 600% en poids.

**67.** Substrat polymère greffé avec du polystyrène selon l'une quelconque des revendications 63 à 66, qui est sous la forme d'une feuille ou d'une pellicule.

**68.** Substrat polymère greffé avec du polystyrène selon l'une des revendications 63 ou 64, qui a été préparé à partir d'un substrat polymère sous la forme d'une bille, d'une pastille, d'un disque, d'un anneau, d'un tube, d'une barre ou d'un filet.

**69.** Substrat polymère greffé avec du polystyrène selon l'une quelconque des revendications 63 à 68, dans lequel le polymère est une polyoléfine.

**70.** Substrat polymère greffé avec du polystyrène selon la revendication 69, dans lequel ladite polyoléfine est le polyéthylène.

**71.** Utilisation, dans une méthode analytique, d'un substrat polymère greffé avec du polystyrène portant des peptides selon l'une quelconque des revendications 17 à 25, en tant que matériau support transparent à la lumière, facilitant la surveillance spectrophotométrique d'une réaction antigène/anticorps, ledit antigène étant un peptide synthétisé sur et restant ancré sur le substrat polymère greffé avec du polystyrène.

**72.** Utilisation selon la revendication 71, ladite surveillance comportant l'emploi d'une technique ELISA.

43

$$\overset{\text{70}}{\text{Boc-Ala-Asp(OBzl)-Lys(2Cl-Z)-Ala-Asp(OBzl)-}}$$

$$\overset{\text{76}}{\text{Val-Asp(OBzl)-Val-Leu-Thr(Bzl)-Lys(2Cl-Z)-}}$$

$$\text{Ala-Lys(2Cl-Z)-Ser(Bzl)-}\overset{\text{84}}{\text{Gln}}\text{-OCH}_2\text{-C}_6\text{H}_4\text{-CH}_2\text{-CO-}$$

$$\text{NH-CH}_2\text{-polystyrene-grafted polyethylene sheet}$$

## Fig. 1

EP 0 433 345 B1

**Fig. 2**

EP 0 433 345 B1

| PEPTIDE | SEQUENCE | | | |
|---------|---|---|---|---|
| | 7 | 12 | 14 | 21 |
| 1 MELITTIN-(7-21) | LYS-VAL-LEU-THR-THR-GLY-LEU- | | PRO-ALA-LEU-ILE-SER-TRP-ILE-LYS-OH | |
| 2 [D-PRO14]MELITTIN-(7-21) | LYS-VAL-LEU-THR-THR-GLY-LEU-D-**PRO**-ALA-LEU-ILE-SER-TRP-ILE-LYS-OH | | | |
| 3 [GLY14]MELITTIN-(7-21) | LYS-VAL-LEU-THR-THR-GLY-LEU- | | **GLY**-ALA-LEU-ILE-SER-TRP-ILE-LYS-OH | |
| 4 [LYS14]MELITTIN-(7-21) | LYS-VAL-LEU-THR-THR-GLY-LEU- | | **LYS**-ALA-LEU-ILE-SER-TRP-ILE-LYS-OH | |
| 5 [ASP14]MELITTIN-(7-21) | LYS-VAL-LEU-THR-THR-GLY-LEU- | | **ASP**-ALA-LEU-ILE-SER-TRP-ILE-LYS-OH | |
| 6 [ASN14]MELITTIN-(7-21) | LYS-VAL-LEU-THR-THR-GLY-LEU- | | **ASN**-ALA-LEU-ILE-SER-TRP-ILE-LYS-OH | |
| 7 [SER14]MELITTIN-(7-21) | LYS-VAL-LEU-THR-THR-GLY-LEU- | | **SER**-ALA-LEU-ILE-SER-TRP-ILE-LYS-OH | |
| 8 [LEU14]MELITTIN-(7-21) | LYS-VAL-LEU-THR-THR-GLY-LEU- | | **LEU**-ALA-LEU-ILE-SER-TRP-ILE-LYS-OH | |
| 9 [PHE14]MELITTIN-(7-21) | LYS-VAL-LEU-THR-THR-GLY-LEU- | | **PHE**-ALA-LEU-ILE-SER-TRP-ILE-LYS-OH | |
| 10 [D-PHE14]MELITTIN-(7-21) | LYS-VAL-LEU-THR-THR-GLY-LEU-D-**PHE**-ALA-LEU-ILE-SER-TRP-ILE-LYS-OH | | | |
| 11 [VAL14]MELITTIN-(7-21) | LYS-VAL-LEU-THR-THR-GLY-LEU- | | **VAL**-ALA-LEU-ILE-SER-TRP-ILE-LYS-OH | |
| 12 [LEU12, GLN14]MELITTIN-(7-21) | LYS-VAL-LEU-THR-THR-**LEU**-LEU- | | **GLN**-ALA-LEU-ILE-SER-TRP-ILE-LYS-OH | |
| 13 [LEU12, LYS14]MELITTIN-(7-21) | LYS-VAL-LEU-THR-THR-**LEU**-LEU- | | **LYS**-ALA-LEU-ILE-SER-TRP-ILE-LYS-OH | |

# Fig. 3

EP 0 433 345 B1

Fig. 4

A_{215} nm

MINUTES

SULFONIUM HOAc SALTS

EP 0 433 345 B1

47